# EUROPEAN PATENT SPECIFICATION

(11) **EP 2 215 184 B1**
(45) Date of publication and mention of the grant of the patent: **20.08.2014**
(21) Application number: 08847778.1
(22) Date of filing: 05.11.2008
(51) Int. Cl.: C07D 413/12, C07D 417/12, C07D 417/14, C09K 11/06

(54) **ORGANIC FLUORESCENT COMPOSITIONS**
ORGANISCHE LEUCHTSTOFFZUSAMMENSETZUNGEN
COMPOSITIONS ORGANIQUES FLUORESCENTES

(30) Priority: 06.11.2007 US 935842
(43) Date of publication of application: 11.08.2010
(73) Proprietor: Honeywell International Inc., Morristown, NJ 07962-2245 (US)
(72) Inventor: POTRAWA, Thomas, 30926 Seelze (DE); SCHULZ, Joachim, 31867 Pohle (DE)
(74) Representative: Stewart, Lucy Caroline
(86) International application number: PCT/US2008/082392
(87) International publication number: WO 2009/061755

(56) References cited:
- EP-B1- 0 832 642
- EP-B1- 0 832 642
- GB-A- 998 368
- US-A1- 2006 002 872
- ANTHONY K ET AL: "SOLID-STATE FLUORESCENT PHOTOPHYSICS OF SOME 2-SUBSTITUTED BENZOTHIAZOLES", JOURNAL OF THE CHEMICAL SOCIETY, PERKIN TRANSACTIONS 2, CHEMICAL SOCIETY. LETCHWORTH, GB, no. 12, 1 January 1984 (1984-01-01), pages 2111-2117, XP009011528, ISSN: 1472-779X, DOI: 10.1039/P29840002111
- JAMIE M. ET AL.: 'Structure.activity relationships of novel antibacterial translation inhibitors: 3,5- Diamino-piperidinyl triazines.' BIOORGANIC & MEDICINAL CHEMISTRY LETTERS. vol. 16, August 2006, pages 5451 - 5456, XP025107192

## Description

### BACKGROUND

### Field of Invention:

This invention relates to organic fluorescent compositions. More particularly, the invention relates to organic fluorescent compositions having a triazine moiety.

### Description of Related Art:

Certain fluorescent compounds are useful for securely marking articles, such as documents, wherein the mark does not reflect incident visible light (i.e., is invisible under normal light), but emits a visible mark when subjected to ultraviolet (UV) light. U.S. Pat. No. 4,504,084, for example, discloses a method for marking originals with a fluorescent compound so that copies can be distinguished from their originals. Other methods of applying fluorescent markers to fibers include those of U.S. Pat. No. 6,217,794, which describes a method for marking fibers and fibrous material with near-infrared compositions using cross-linking agents.

Security paper is typically produced by incorporating microfibers marked with an invisible fluorescent agent directly into the paper. For such paper, microfibers constructed of viscose rayon are preferable to other polymer fibers, such as polyamines, because viscose rayon fiber are easier to incorporate into the paper, are less stiff, and, with respect to common inks, have bonding characteristics similar to those of paper. Yet few fluorescent agents are suitable for marking viscose rayon fibers, particularly for security applications. For example, inorganic fluorescent pigments may be applied to viscose rayon fiber, but typically have low luminous intensities making them unreliable for most security applications. Most organic fluorescent pigments that are thermally, chemically, and photo stable do not easily bond directly to cellulose, and instead require the use of a binding agent. Unfortunately, binding agents in a dye solution can lead to a clumping of the viscose rayon fibers during the marking process. Thus, it is desirable to find organic fluorescent compounds that can bond directly to viscose rayon fibers.

Certain triazines are known to be active when exposed to UV light. For example, U.S. Pat. No. 3,167,565 describes an organic triazine compound useful as an optical brightener that purportedly has an affinity for binding to fiberous substrates including cellulose, nylon, and wool. However, the compounds described in this patent, notably: 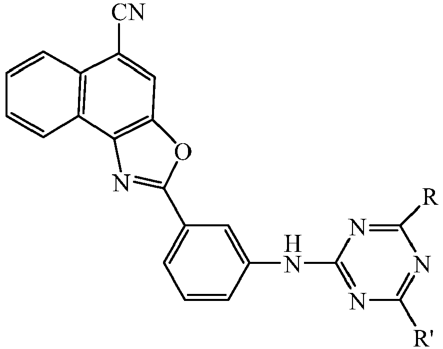 wherein R and R' are an alkoxy, allyloxy, phenoxy, tertiary amine, etc., are optical brighteners (i.e., a dye that absorbs light in the ultraviolet and violet regions of the electromagnetic spectrum and re-emit light in the blue region, thereby causing a "whitening" effect, making materials look less yellow and increasing the overall amount of light reflected to the eye). While brighteners are used in many papers, especially high brightness papers, paper used for banknotes and other secure applications does not contain optical brighteners since the brightener would interfere with common methods for detecting forged notes via a check for specific fluorescence.

Other triazine compounds are also known to be active when exposed to UV light, but these compounds are notably characterized as UV absorbers, i.e., compounds that absorb light in the ultraviolet region of the electromagnetic spectrum, but do not re-emit the absorbed energy as visible light. The general structure of a UV absorbent molecule allows it to absorb a broad spectrum of high-energy ultraviolet rays and then release the absorbed energy as lower-energy rays (generally, at an unspecific wavelength or at a wavelength outside of the visible band). Such compounds are typically used in sunscreens and other topically applied compositions to reduce sunburn and other skin damage due to exposure to UV radiation.

Examples of UV absorbent triazine compounds include those described in EP 0 832 642, such as the following molecule: 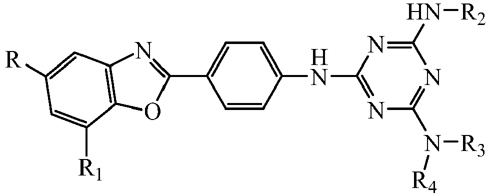 which purportedly is useful as a UV filter in cosmetic or dermatological compositions; those described in US 2006/0002872 such as the following molecule: 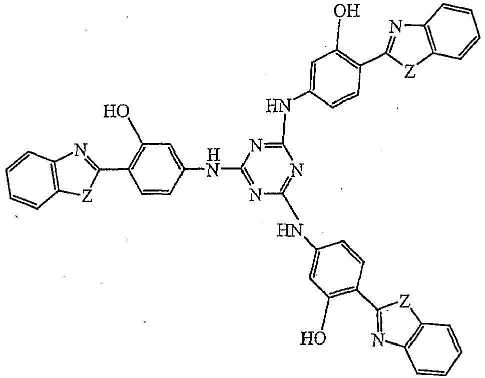 wherein Z is oxygen or sulfur, which are also purportedly useful in topically applied cosmetic or dermatological photoprotective compositions; and those described in Swiss Pat. No. CH 439283 and German Pat. No. DE 1205970, including those having absorbing triazine compounds having a substituent moiety of: 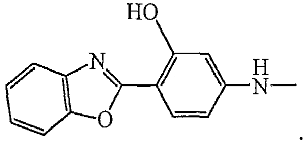 GB 998 368 discloses compounds of formula 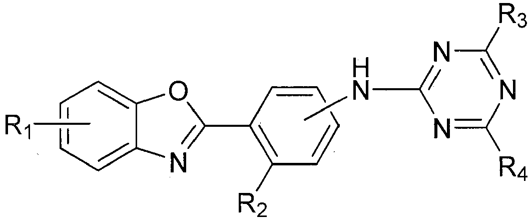 which are useful as light filters in a variety of different applications.
Anthony et al (J. Chem. Soc; Perkin, Trans. II; 1984, p. 2111-2117) discloses that certain compounds having the following structure: 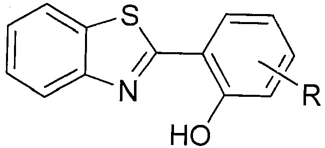 fluoresce towards the red region of the visible spectrum.

Thus, there remains a need for fluorescent compounds useful as security markers that can be easily covalently bonded to viscose rayon microfibers.

### SUMMARY OF THE INVENTION

Applicants have found triazine compounds with a single or double substituent moiety of the formula: 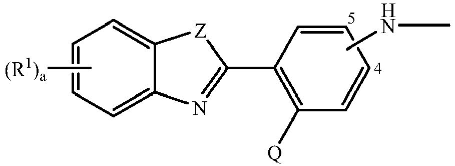 wherein R¹ is independently a C₁ - C₈ branched or straight chain alkyl; a is 0, 1, 2, 3, or 4; Z is oxygen or sulfur; and Q is an acidic hydrogen moiety selected from OH, NH₂, NHR², wherein R² is a C₁ - C₈ branched or straight chain alkyl, fluoresce with a high luminous intensity at specific visible wavelengths. These compounds are stable and are particularly adaptable for use as a pigment or dye in inks. Moreover, some of these compounds can directly bond to a cellulose or cellulose derivative substrate.

The fluorescent property of these compounds is surprising since certain other triazine compounds having substituents of the same or similar formula do not fluoresce, but instead function as UV absorbents. As used herein, the term "fluorescent compound" means a compound having a functional group or moiety which will molecularly absorb photonic energy of a specific UV wavelength and subsequently re-emit of at least a portion of the absorbed energy as photonic energy at a different (but equally specific) wavelength within the visible light range. The functional group or moiety responsible for the compound's fluorescent property is referred to as its fluorophore. The intensity and wavelength of the energy emitted by the fluorescent compound depend on the fluorophore's molecular structure (i.e., different isomers of a compound tend to luminesce at different wavelengths which produces different colors).

Accordingly, an aspect of the present invention is a composition comprising an organic fluorophore having a structure according to Formula I: 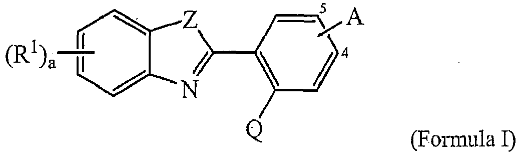 wherein:
R¹ is independently a C₁ - C₈ branched or straight chain alkyl;
a is 0, 1, 2, 3, or 4;
Z is S;
Q is an acidic hydrogen moiety selected from OH, NH₂, NHR², wherein R² is a C₁-C₈ branched or straight chain alkyl; and
A is an amino-triazine moiety bonded to the 4^{th} or 5^{th} carbon of the phenol or aniline ring, said amino-triazine moiety having a structure according to Formula II: 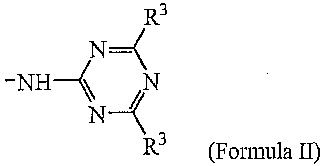
   wherein R³ is independently
   a halogen,
   a benzothiazole having a structure according to Formula X: 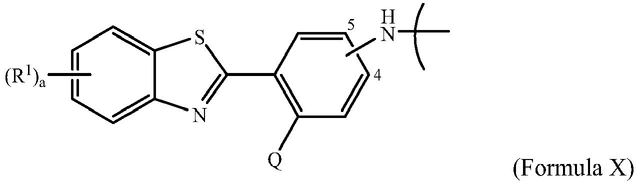
      wherein Q, R¹, and a are defined as above,
   a benzooxazole having a structure according to Formula XI: 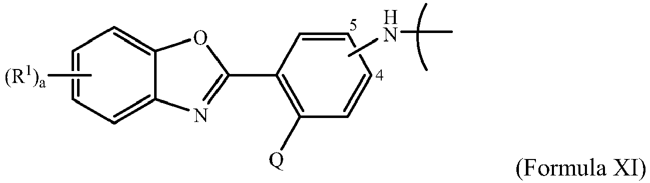
      wherein Q, R¹, and a are defined as above,
   or a tertiary amine having a structure according to Formula III: 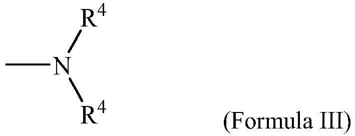
      wherein R⁴ is independently a C₁ - C₂₀ linear or branched, substituted or unsubstituted alkyl, alkyoxy, hydroxyalky, or carboxylic acid, or a member of substituted or unsubstituted C₁ - C₂₀ heterocyclic alkyl having the N atom positioned between the two R⁴ groups, and optionally having additional N, O, or S heteroatoms;
provided that no more than one R³ is a halogen and that no more than one R³ is said benzothiazole or said benzooxazole.

Preferably, the R³ substituents are chosen so as to enhance physical properties (e.g., solubility, stability, etc.) of the compound other than its luminescent properties (e.g., color, intensity, etc.). For example, if the desired composition is an ink, then the R³ substituents can be chosen to facilitate the fluorescent compound's solubility in a dye-suitable solvent or its uniform dispersion as a pigment in a resin.

According to another aspect of the invention, provided is a method for marking an article comprising contacting at least a portion of the article with the above-described composition wherein the contacting produces a detectible residue on the portion of the article.

Applicants have also found that many of these fluorescent compounds can be synthesized from a [4,6-dihalo-s-triazine-2-yl]amine having a benzothiazolyl phenol, benzothiazolyl aniline, or benzothiazolyl alkylaniline moiety. Thus, according to another aspect of the invention, provided is a fluorescent compound having a structure according to Formula IV: 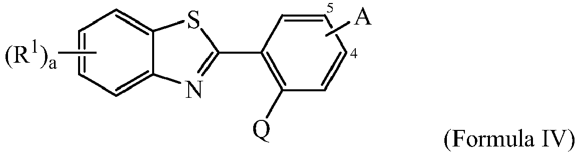 wherein:
R¹ is independently a C₁ - C₈ branched or straight chain alkyl;
a is 0, 1, 2, 3, or 4;
Q is an acidic hydrogen moiety selected from OH, NH₂, NHR², wherein R² is a C₁ - C₈ branched or straight chain alkyl; and
A is an amino triazine moiety bonded to the 4^{th} or 5^{th} carbon of the phenol or aniline ring, said amino triazine moiety having a structure according to Formula V: 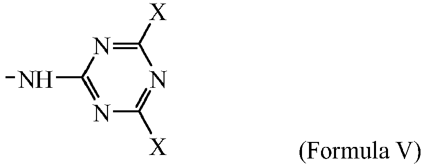
   wherein X is independently a halogen, preferably Br or Cl, and more preferably Cl.

Applicants have further found that compounds of Formula IV, particularly the compounds having chlorine substituents, readily bond directly to cellulose and cellulose derivatives (e.g., viscose rayon). The compound's propensity for bonding directly to cellulose and cellulose-derived substrates is particularly advantageous because most organic fluorescent compounds require a binding agent to adhere to cellulose substrates. This result is also surprising in view of the fact that others in the art suggest substituting the chlorine atoms on -[4,6-dichloro-s-triazin-2-yl]- compounds with oxy- or amino- functional groups to produce a final product having an affinity for cellulose. (See, US Pat. No. 3,167,565). Accordingly, another aspect of the invention provides an article of manufacture comprising a synthetic polymeric fibers, cellulose, or cellulose derivative substrate covalently bonded to an organic fluorophore having a structure according to Formula VI: 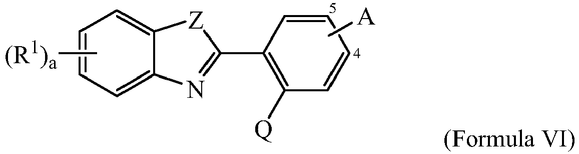 wherein:
R¹ is independently a C₁ - C₈ branched or straight chain alkyl;
a is 0, 1, 2, 3, or 4;
Z is O or S;
Q is an acidic hydrogen moiety selected from OH, NH₂, NHR², wherein R² is a C₁ - C₈ branched or straight chain alkyl; and
A is an amino-triazine moiety bonded to the 4^{th} or 5^{th} carbon of the phenol or aniline ring, said amino-triazine moiety having a structure according to Formula VII: 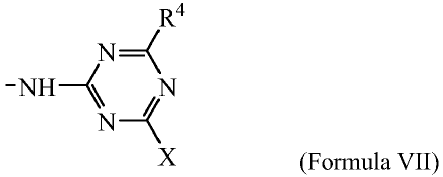
   wherein X is a halogen and R⁴ is a covalent bond joining said organic fluorophore and said substrate.

Also provided is a method for producing a fluorescent fiber comprising (a) providing a cellulose or cellulose derivative fiber, preferably viscose rayon, and (b) contacting the fiber with a fluorescent compound under conditions effective to covalently bond the fluorescent compound to the fiber, wherein the fluorescent compound has a structure according to Formula VIII: 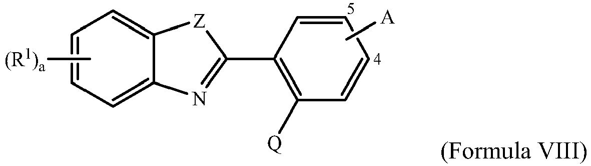 wherein:
R¹ is independently a C₁ - C₈ branched or straight chain alkyl;
a is 0, 1, 2, 3, or 4;
Z is O or S;
Q is an acidic hydrogen moiety selected from OH, NH₂, NHR², wherein R² is a C₁ - C₈ branched or straight chain alkyl; and
A is an amino-triazine moiety bonded to the 4^{th} or 5^{th} carbon of the phenol or aniline ring, said amino-triazine moiety having a structure according to Formula IX: 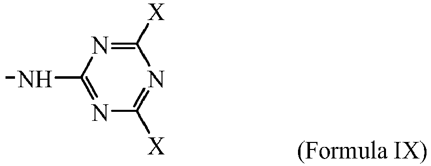
   wherein X is independently a halogen, preferably Cl.

### DETAILED DESCRIPTION OF PREFERRED EMBODIMENTS OF THE INVENTION

Compositions of the present invention can be of several types including fluorescent dyes and pigments, as well as inks, paints, or other colorants such as printer toner into which the fluorescent dye or pigment is incorporated. These compositions comprise an organic fluorophore having a structure according to Formula I: 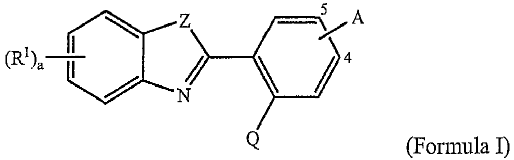 wherein:
R¹ is independently a C₁ - C₈ branched or straight chain alkyl;
a is 0, 1, 2, 3, or 4;
Z is S;
Q is an acidic hydrogen moiety selected from OH, NH₂, NHR², wherein R² is a C₁ - C₈ branched or straight chain alkyl; and
A is an amino-triazine moiety bonded to the 4^{th} or 5^{th} carbon of the phenol or aniline ring, said amino-triazine moiety having a structure according to Formula II: 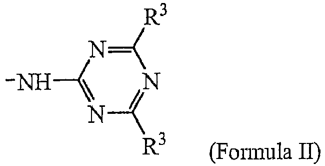
   wherein R³ is independently
   a halogen,
   a benzothiazole having a structure according to Formula X: 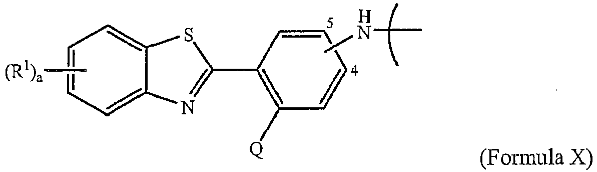
      wherein Q, R¹, and a are defined as above,
   a benzooxazole having a structure according to Formula XI: 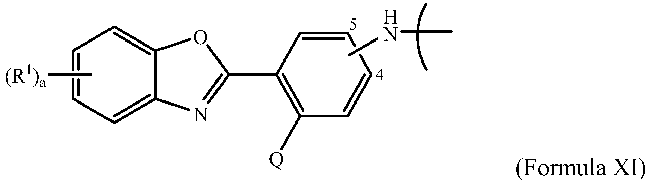
      wherein Q, R¹, and a are defined as above,
   or a tertiary amine having a structure according to Formula III: 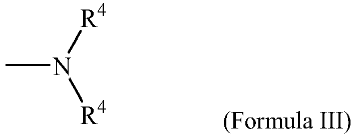
      wherein R⁴ is independently a C₁ - C₂₀ linear or branched, substituted or unsubstituted alkyl, alkyoxy, hydroxyalky, or carboxylic acid, or a member of substituted or unsubstituted C₁ - C₂₀ heterocyclic alkyl having the N atom positioned between the two R⁴ groups, and optionally having additional N, O, or S heteroatoms;
provided that no more than one R³ is a halogen and that no more than one R³ is said benzothiazole or said benzooxazole.

In general, the luminescent intensity and wavelength (i.e., color) of the photons emitted by the fluorescent compound depend on the fluorophore's molecular structure. Even different isomers and structuraly similar compounds can produce prominent distinctions in color. For example, each of the following compounds fluoresce to give a different color: 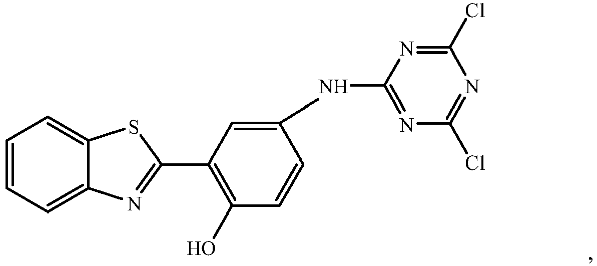 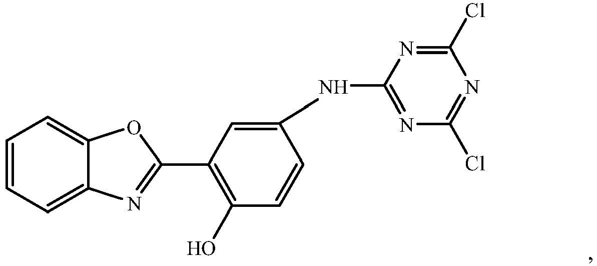 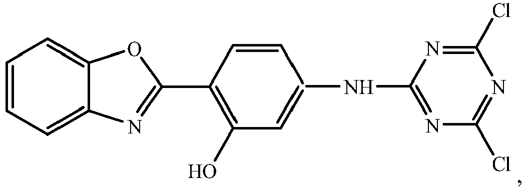 and 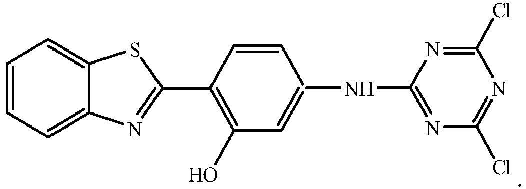 More specifically, 2-(2-Benzothiazolyl)-5-[[4,6-dichloro-s-trazin-2-yl]amino]-phenol fluoresces yellow (555 nm) when exposed to UV radiation. In contrast, 2-(2-Benzoxazolyl)-5-[[4,6-dichloro-s-trazin-2-yl]amino]-phenol; 2-(2-Benzoxazolyl)-4-[[4,6-dichloro-s-trazin-2-yl]amino]-phenol; and 2-(2-Benzothiazolyl)-4-[[4,6-dichloro-s-trazin-2-yl]amino]-phenol fluoresce green (525 nm), blue (475 nm), and blue-green (500 nm), respectively, when exposed to similar UV radiation.

In general, the R³ moieties of the fluorophore of Formula I do not negate the compound's fluorescent property, provided that they are selected from the moieties defined above. Preferably, the selection of specific R³ substituents is primarily directed to enhancing the physical properties of the compound other than its luminescence. For example, if the desired composition is a pigment- or dye-based ink, the R³ substituents of the fluorophore can be chosen to facilitate the fluorescent compound's solubility in a solvent or the fluorescent compound's uniform dispersion in a resin. Here, a distinction is made between pigments, which are typically solid and insoluble in their carrier medium (resulting in a suspension), and a dye, which either is itself a liquid or is generally soluble in its solvent (resulting in a solution).

In certain embodiments, the fluorophore functions as a fluorescent pigment. For such embodiments, the R³ substituents of the fluorophore are preferably independently selected from the group consisting of chlorine, substituted or unsubstituted piperidyl, substituted or unsubstituted pyrrolidyl, substituted or unsubstituted piperazin-1-yl, substituted or unsubstituted morpholin-4-yl, provided that no more than one R³ is chlorine. More preferably R³ is independently selected from chlorine, piperid-1-yl, piperazin-1-yl, 4-methyl-piperazin-1-yl, and morpholin-4-yl, provided that no more than one R³ is chlorine.

In some embodiments, the composition is a pigment and consists essentially of the fluorophore (i.e., the composition does not contain solvents, carriers, or other compounds that would materially affect the composition's luminosity or physical appearance). In certain other embodiments, the composition is a pigment and comprises the fluorophore and further comprises one or more solid additives such as a stabilizing agent, particulate matter, and processing aid including solid lubricants (e.g., magnesium stearate).

In some embodiments, the composition is a pigment-based ink and comprises the fluorophore as well as one or more carriers in the form a suspension, paste, or gel. Examples of preferred carriers include water, casein, gum arabic, and resins such as modified natural rosins including gum rosin, wood rosin, and tall oil rosin, and synthetic functionalized rosins including polymerized or dimerized rosin and their esters, metallic resinates, phenolic and/or maleic/fumaric modified rosins and their esters, ester gums, cyclic and dicyclic unsaturated hydrocarbon rosins. Such suspensions may optionally comprise one or more additives selected from the group consisting of lubricants, solubilizers, coalescent solvents, biocides, anti-foaming agents, emulsifying agents, particulate matter, colorants, drying agents, chemical stabilizers, photostabilizers, and other materials that function to modify the solubility, viscosity, flow, color, and/or thickness of the composition and/or its appearance after it dries. The suitability of a carrier or additive to a particular composition must be determined on a case-by-case basis, but is readily determinable by one skilled in the art.

In certain embodiments, the fluorophore functions as a fluorescent dye. For such embodiments, the R³ substituents of the fluorophore can, for example, be independently selected from the group consisting of chlorine, *N*-(C₁-C₈ alkyl)-*N*-(C₁-C₈ alkyl) amine, or *N*-(C₁-C₈ carboxylic acid)-*N*-(C₁-C₈ carboxylic acid) amine, with N-(propyl)-N-(propyl) amine being particularly preferred due to the resulting composition's solubility in methyl-ethyl-ketone.

In some embodiments, the composition is a dye and consists essentially of the fluorophore. That is, the composition is a liquid itself and does not contain solvents or other compounds that would materially affect the composition's luminosity or physical appearance.

In some embodiments, the composition is a dye-based ink and comprises the fluorophore and one or more solvents (in which the dye is at least partially miscible) to form a solution. Preferred solvents in inks include those that are compatible with paper and related goods, and/or can be utilized in a printing or other marking process. Examples of suitable solvents include C₁-C₈ alcohols, glycols, ether alcohols, sulfoxides, amines, amides, C₃-C₁₂ alkyl ketones, ethers, esters, nitriles, branched or linear alkyls, cycloalkyls, aromatics, polyvinyl alcohols, and ethylene oxide phenols, and mixtures thereof. A particularly preferred solvent is methyl-ethyl-ketone.

Dye-based inks of the present invention may optionally include additives including, but not limited to, lubricants, solubilizers, emulsifying agents, particulate matter, colorants, drying agents, chemical stabilizers, photostabilizers, and other materials that function to modify the solubility, viscosity, flow, color, and or thickness of the solution and/or appearance of the dye when it is applied to an article and dried. The suitability of one or more of these components as an additive to an ink composition must be determined on a case-by-case basis but is readily apparent to one skilled in the art.

In some embodiments, the composition comprises a fluorophore that is dissolved, suspended, and/or stabilized in a cosmetically and/or dermatologically acceptable oil. Such compositions, which are typically utilized as cosmetics, may optionally comprise other ingredients that are cosmetically and/or dermatologically acceptable.

Compounds of Formula I having a benzothiazolyl moiety can be derived from a precursor having a structure according to Formulae IV or V: 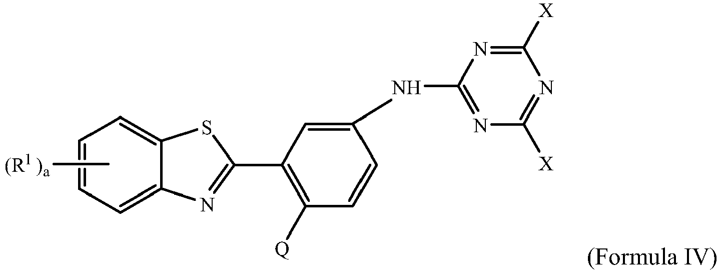 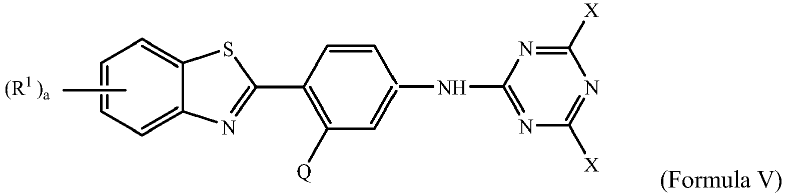 wherein:
R¹ is independently a C₁ - C₈ branched or straight chain alkyl;
a is 0, 1, 2, 3, or 4;
Q is an acidic hydrogen moiety selected from OH, NH₂, NHR², wherein R² is a C₁ - C₈ branched or straight chain alkyl; and
X is independently a halogen, preferably Br or Cl, and more preferably Cl.

For example, 2-(2-Benzothiazolyl)-5-[4,6-dichloro-s-triazin-2-yl]amino]-phenol can be reacted with morpholine in the liquid phase to produce 2-(2-Benzothiazolyl)-5-[[4-chloro-6-morpholin-s-triazin-2-yl]amino]-phenol and also 2-(2-Benzothiazolyl)-5-[[4,6-dimorpholin-s-triazin-2-yl]amino]-phenol, depending on the amount of morpholine available as a reactant. This synthesis method involves the nucleophilic substitution of one or more of the chlorine atoms with a corresponding number of morpholine moieties. Similarly, 2-(2-Benzothiazolyl)-5-[4,6-dichloro-s-triazin-2-yl]amino]-phenol can undergo a nucleophilic substitution reaction with piperidine, 4-methylpiperidne, piperazine, 4-methylpiperazine, or propylene to form other compounds of Formula I.

The 2-(2-Benzothiazolyl)-5-[4,6-dichloro-s-triazin-2-yl]amino]-phenol precursor compound can be synthesized by reacting equal molar amount of (hydroxy-5-aminophenyl)-benzthiazol and 1,3,5-trichlorotriazine in the liquid phase and in the presence of an acid scavenger such as sodium bicarbonate.

In addition to serving as a precursor for other fluorescent compounds, the compounds of Formulae IV and V can also serve as fluorescent markers themselves due, in part, to their fluorescent properties and their ability to bond directly to cellulose, cellulose derivatives, and related polymeric substrates. These substrates can generally be of any desired physical form, including fibers, yarns, threads, fabrics, and films, with fibers being particularly preferred. These substrates can be fashioned into a variety of articles including paper products; packaging materials including cardboard; book covers; fabrics used in suits, dresses, sportswear, shirts, ties, and other garments; fabrics for medical uses; filtration materials; nonwovens; adhesive tapes; and the like.

Without being bound to any particular theory, it is believed that reacting cellulose with the organic fluorophore results in the combined dehalogenation of the fluorophore compound and dehydration of the cellulose substrate to form an ether linkage between the fluorophore and cellulose. Accordingly, a preferred embodiment of the invention is an article of manufacture comprising a substrate selected from cellulose, cellulose derivatives, and related polymeric materials covalently bonded to an organic fluorophore having a structure according to Formula VI: 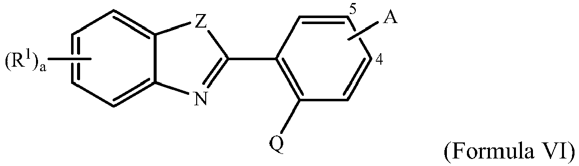 wherein:
R¹ is independently a C₁ - C₈ branched or straight chain alkyl;
a is 0, 1, 2, 3, or 4;
Z is O or S;
Q is an acidic hydrogen moiety selected from OH, NH₂, NHR², wherein R² is a C₁ - C₈ branched or straight chain alkyl; and
A is an amine-substituted triazine moiety bonded to the 4^{th} or 5^{th} carbon of the aromatic ring, said amine-substituted triazine moiety having a structure according to Formula VII: 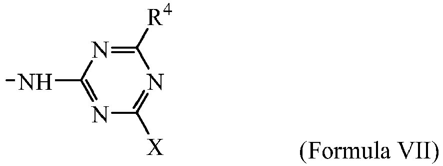
   wherein X is a halogen, preferably Cl, and R⁴ is a covalent bond joining said organic fluorophore and said substrate.

Preferred substrates include cellulose fibers, such as cotton, and cellulose derivative fibers, such as viscose rayon, with viscose rayon being particularly preferred. The fiber can be of any practical length and can have a round or non-round cross-section. These fibers can be woven, knitted, or napped into a textile or spun into a nonwoven fabric either before or after bonding to the fluorophore. Moreover, such a fabric or textile may comprise one or more filaments constructed of a material other than cellulose or cellulose derivatives.

The fluorescent fibers are produced by a method comprising the steps: (a) providing a cellulose or cellulose derivative fiber, and (b) contacting the fiber with a fluorescent compound under conditions effective to covalently bond the fluorescent compound to the fiber, wherein the fluorescent compound has a structure according to Formula VIII: 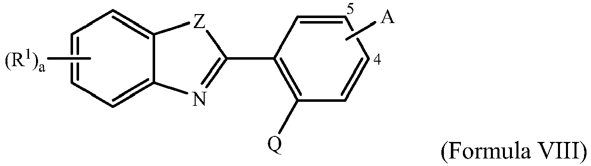 wherein:
R¹ is independently a C₁ - C₈ branched or straight chain alkyl;
a is 0, 1, 2, 3, or 4;
Z is O or S;
Q is an acidic hydrogen moiety selected from OH, NH₂, NHR², wherein R² is a C₁ - C₈ branched or straight chain alkyl; and
A is an amino-triazine moiety bonded to the 4^{th} or 5^{th} carbon of the phenol or aniline ring, said amine-substituted triazine moiety having a structure according to Formula IX: 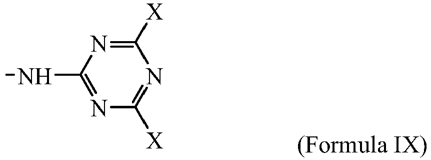
   wherein X is independently a halogen, preferably Cl.

According to the present invention, the cellulose or cellulose derivative substrate may be marked with a fluorescent compound by dissolving or suspending the compound in an organic solvent or carrier and then applying the resultant solution or suspension to the substrate. Contact between the fluorophore solution or suspension and the substrate can be performed continuously or batch-wise using techniques well known to those skilled in the art. For example, the contacting step may comprise spraying or dropping the fluorophore solution or suspension onto the substrate; immersing the substrate in a bath containing the fluorophore solution or suspension; contacting the substrate with a continuous flow of the fluorophore solution or suspension; or some combinations of these techniques. For some applications, it is desirable that the fluorophore solution or suspension contact substantially all the substrate's surface to provide uniform coverage of the resultant fibers while in other applications only a portion of the substrate's surface need be contacted.

The duration, temperature, and pressure of the contacting step is not particularly limited, provided that the process is conducted in an environment and for an amount of time to effectively produce a detectable mark on the substrate. Preferably, the contacting step is conducted at a temperature of about 25 to about 100 °C, more preferably from about 50 to about 100 ° C, and even more preferably from about 60 to about 65° C, a pressure of about 0.1 to about 10 atmospheres, with ambient pressure being preferred for convenience, safety, and economy. Contact time is preferably from about 1 second to about 10 hours, more preferably from about 10 minutes to about 4 hours, and even more preferably from about 45 to about 90 minutes.

Preferably, the solvent or carrier medium is removed via one or more washing, filtering, and/or evaporation processes subsequent to contacting the substrate with the fluorophore. Suitable evaporation techniques include treatment with an appropriate drying step such as heating in a tow dryer or on heated rolls, etc., which are equipped with suitable partial vacuum and/or ventilation means. Advantageously, vacuum drying further removes any residual solvent or carrier volatiles from the surface of the substrate. The overall heating or drying time of each type of fiber or application can be determined readily by those skilled in the art based on the particular solvent used and the drying process conditions (e.g., temperature and volumetric flow of air).

Certain substrates, particularly viscose rayon fibers, marked with the fluorophore can subsequently be incorporated into an article, such as paper, to form a security mark. In other embodiments, an article is marked with the fluorophore via a printing process. For example, the fluorescent compound can be applied as an ink or toner to a substrate, such as paper goods or packaging materials, via a printing process such as ink-jet printing, thermal printing, piezo printing, laser printing, offset lithography, relief printing, screen printing, dye transferring, or pad printing.

According to the invention, the fluorophores can be utilized in a method of securely marking an article comprising contacting at least a portion of the article with the fluorophore or related composition comprising an organic fluorophore having a structure according to formula I 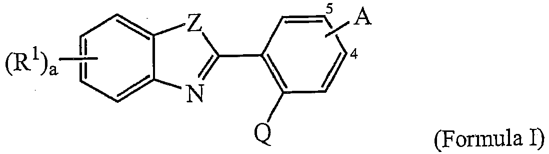 wherein:
R¹ is independently a C₁ - C₈ branched or straight chain alkyl;
a is 0, 1, 2, 3, or 4;
Z is O or S;
Q is an acidic hydrogen moiety selected from OH, NH₂, NHR², wherein R² is a C₁ - C₈ branched or straight chain alkyl; and
A is an amino-triazine moiety bonded to the 4^{th} or 5^{th} carbon of the phenol or aniline ring, said amino-triazine moiety having a structure according to Formula II: 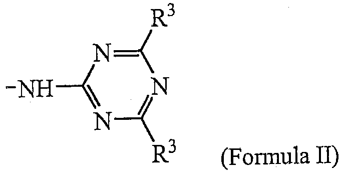
   wherein R³ is independently
   a halogen,
   a benzothiazole having a structure according to Formula X: 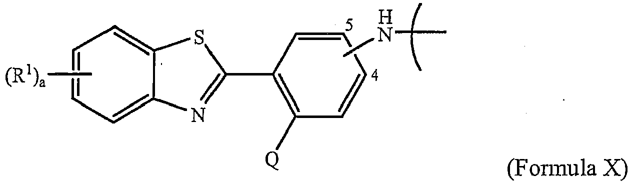
      wherein Q, R¹, and a are defined as above,
   a benzooxazole having a structure according to Formula XI: 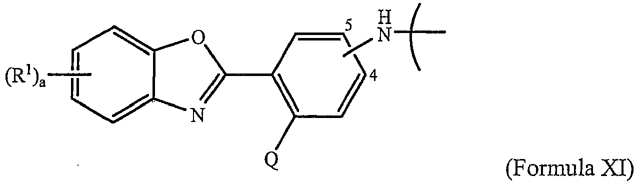
      wherein Q, R¹, and a are defined as above,
   or a tertiary amine having a structure according to Formula III: 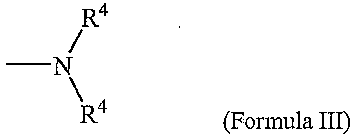
      wherein R⁴ is independently a C₁ - C₂₀ linear or branched, substituted or unsubstituted alkyl, alkyoxy, hydroxyalky, or carboxylic acid, or a member of substituted or unsubstituted C₁ - C₂₀ heterocyclic alkyl having the N atom positioned between the two R⁴ groups, and optionally having additional N, O, or S heteroatoms;
provided that no more than one R³ is a halogen and that no more than one R³ is said benzothiazole or said benzooxazole wherein the contacting produces a detectible residue on the portion of the article and wherein said article comprises a substrate selected from synthetic polymer fibres, cellulose and cellulose derivatives. The contacting step may be performed on the article as a whole or on a portion of the article that is subsequently incorporated into the whole article. For example, the fluorophore composition may printed as an image directly on a paper or may be bonded to a fiber which is subsequently incorporated into a paper. Both methods are particularly useful in marking secure papers such as identity cards, bank notes, vouchers, tickets, checks, coupons, tags, labels, and certificates, and metering application such as such as postal payment.

The mark imparted on the article is preferably detectible using an automated detector that is responsive to fluorescent emissions. When the original security marking image is illuminated with an ultraviolet light source, the security image is bright against the low fluorescence background of the paper, but under visible light has no readily detectible appearance. A copied security document, on the other hand, has no detectible security markings when exposed to UV radiation. Thus, copied documents can be distinguished from originals, but only by using specialized equipment.

In certain preferred embodiments, the security markings are compatible with visible inks so as to provide fluorescent security markings that are coincident with visible images, making them very difficult to detect and copy and, preferably are also capable of providing unique "fingerprints" that can be identified and traced forensically. A further aspect of the present invention relates to the use of a fluorophore having a structure according to Formula I as a security marker 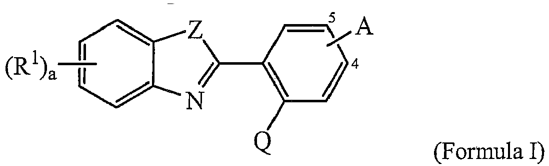 wherein:
R¹ is independently a C₁ - C₈ branched or straight chain alkyl;
a is 0, 1, 2, 3, or 4;
Z is O or S;
Q is an acidic hydrogen moiety selected from OH, NH₂, NHR², wherein R² is a C₁ - C₈ branched or straight chain alkyl; and
A is an amino-triazine moiety bonded to the 4^{th} or 5^{th} carbon of the phenol or aniline ring, said amino-triazine moiety having a structure according to Formula II: 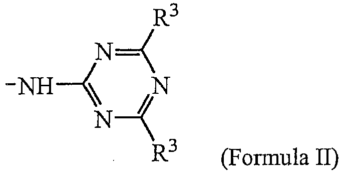
   wherein R³ is independently
   a halogen,
   a benzothiazole having a structure according to Formula X: 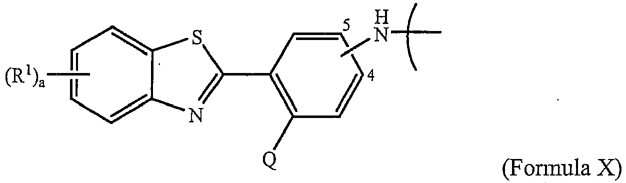
      wherein Q, R¹, and a are defined as above,
   a benzooxazole having a structure according to Formula XI: 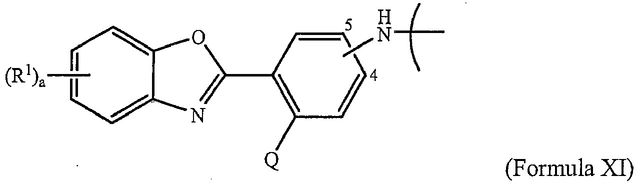
      wherein Q, R¹, and a are defined as above,
   or a tertiary amine having a structure according to Formula III: 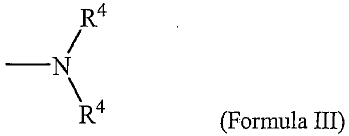
      wherein R⁴ is independently a C₁ - C₂₀ linear or branched, substituted or unsubstituted alkyl, alkyoxy, hydroxyalky, or carboxylic acid, or a member of substituted or unsubstituted C₁ - C₂₀ heterocyclic alkyl having the N atom positioned between the two R⁴ groups, and optionally having additional N, O, or S heteroatoms;
provided that no more than one R³ is a halogen and that no more than one R³ is said benzothiazole or said benzooxazole

### EXAMPLES

Certain aspects of the present invention are further illustrated, but are not limited by, the following examples. For these examples, fluorescent intensity was measured using a Minolta luminance meter under 365nm excitation. Emission maximum and color corrodinates were determined using a Photofluoroescence Spectrometer Fluorolog manufactured by HORIBA Jobin Yvon, Inc.

### Examples 1-4 and Comparative Example 1: Dying Rayon Fibers with Fluorescent Dichlorotriazine Derivatives

These examples illustrate embodiments of the invention wherein viscose rayon fibers are dyed with a dichlorotriazine compounds.

Approximately 10 g of dried rayon fibers with 28 dtex diameter and 3 mm length were added to about 600-700 ml of water in a 1L beaker equipped with a magnetic stirrer. The water and fibers were heated to a temperature of about 60-65°C and then about 50 g sodiumsulfate were added and for further 15 min stirred at about 60 °C.

A dichlorotriazine dye solution was prepared by adding approximately 0.1 g of one of the dichlorotriazine dyes listed in Table 1 below to about 3.5 g of N-methyprynolidon under slightly warming to form a clear solution.

The dye solution was added slowly to the fiber - water mixture, to form a milky dispersion. After about 45 min at 60-65°C, about 1g of sodium carbonate was added and stirred for further 60 min at this temperature. After this time the fibers the liquid portion of the fiber mixture was removed via suction filtration.

The dyed fibers were then washed with about 1.5 L of water and then stirred at room temperature in 600 ml of water. About 0.5 ml of acetic acid was subsequently added and stirred for about 15-20 min and then removed by suction filtration. The remaining fibers where then washed again with 300 ml of water and then dried at 50 °C to yield colorless to off-white microfibers.

The dyed microfibers were tested for emission wavelengths and compared to the luminescent emission of comparative compound identified in Table 1.

**TABLE 1**

| *Ex.* | *Fluorescent Compound* | *Color Coordinate (X*/*Y)* | *Fluorescence Maximum* |
|---|---|---|---|
| 1 | 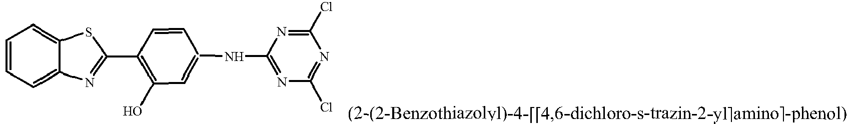 | 0.238 / 0.481 | 500 nm (blue-green) |
| 2 | 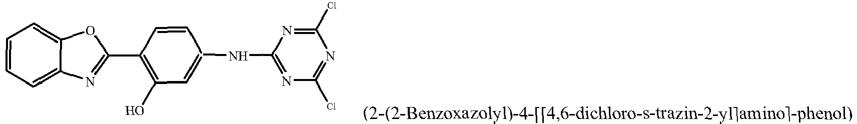 | 0.167 / 0.256 | 475 nm (blue) |
| 3 | 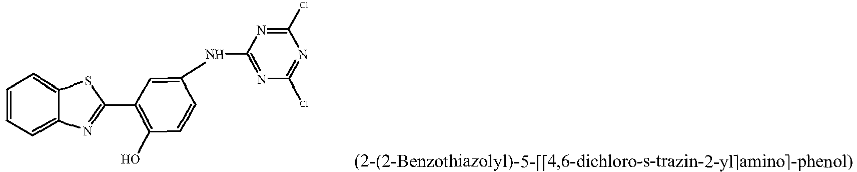 | 0.422 / 0.516 | 555 nm (yellow) |
| 4 | 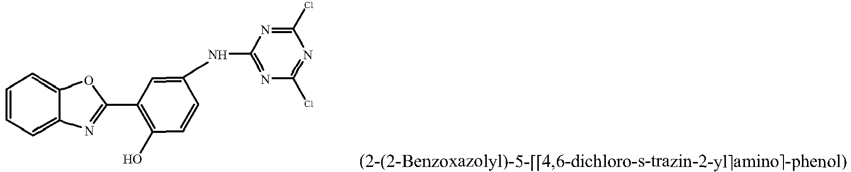 | 0.318 / 0.543 | 525 nm (green) |
| Comp Ex. 1 | 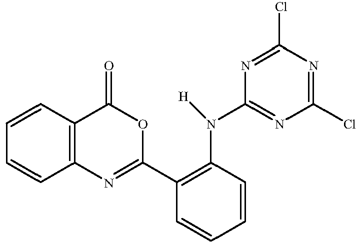 | 0.220 | Dull bluish |

### Example 5: Preparation of 2-(2-Benzothiazolyl)-5-[[4,6-dichloro-s-triazin-2-yl] amino]-phenol

This example illustrates the preparation of the following fluorescent compound of the present invention:

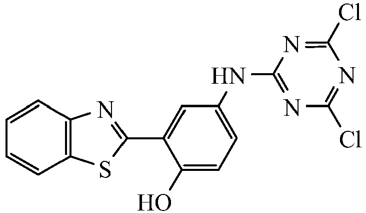

Approximately 2.43 g (0.01 mol) of (hydroxy-5-aminophenyl)-benzthiazol, 1.84 g (0.01 mol) of 1,3,5-trichlorotriazine, and 0.85 g (0.01 mol) of sodium bicarbonate were stirred for 24 h at room temperature in 2-Butanone. The resulting precipitate was filtered by suction, washed with 2-Butanone and water, and then dried at 85 °C.

Analysis of the precipitate showed a yield of 2.5 g (64%) of grey fine needles of 2-(2-Benzothiazolyl)-5-[4,6-dichloro-s-triazin-2-yl]amino]-phenol having about a 99% purity. Differential Thermal Analysis (DTA) demonstrated an endothermic peak of 252° C.

### Example 6: Preparation of 2-(2-Benzothiazolyl)-5-[[4-chloro-6-morpholin-s-triazin-2-yl]amino]-phenol

This example illustrates the preparation of the following fluorescent compound of the present invention:

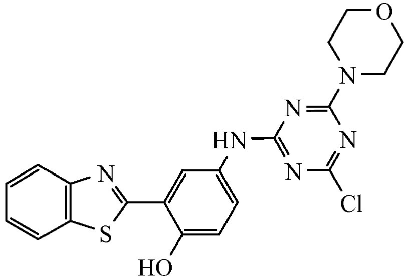

Approximately 20g (0.051 mol) of 2-(2-Benzothiazolyl)-5-[4,6-dichloro-s-triazin-2-yl]amino]-phenol was added to about 500 ml of butanone in a 1L three-neck round flask equipped with a drop funnel, mechanical stirrer and thermometer. The mixture was stirred at room temperature until it was nearly a clear solution. Approximately 8.95 g (0.10 mol) of morpholine was added to the solution via the drop funnel over a period of approximately 15 min. The drop funnel was rinsed with 10 ml Butanaone and the reaction mixture was stirred for another 48 h at room temperature. After this time the off-white precipitate was filtered by suction, washed with cold butanone and then two times suspended in 700 ml water. The solid was again filtered by suction and dried.

Analysis of the precipitate showed a yield of 22.2 g (98.1%) off-white powder of 2-(2-Benzothiazolyl)-5-[4-chloro-6-morpholin-s-triazin-2-yl]amino]-phenol. The compound demonstrated an maximum emission value at 565 nm (green-yellow), a DTA peak of 262 °C, an intensity of fluorescence of 116 (1% draw down, [cd/m²]), and CIE color coordinates of 0.504 and 0.492 (X and Y, respectively).

### Example 7: Preparation of 2-(2-Benzothiazolyl)-5-[[4,6-dimorpholin-s-triazin-2-yl] amino]-phenol

This example illustrates the preparation of the following fluorescent compound of the present invention:

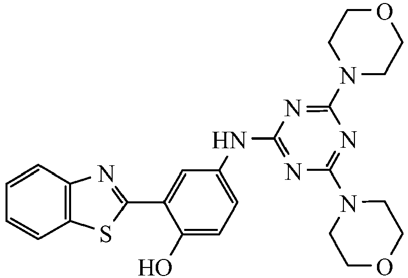

The procedure of Example 6 was repeated, except that approximately 17.86 g (0.205 mol) of morpholine was added to the 2-(2-Benzothiazolyl)-5-[4,6-dichloro-s-triazin-2-yl]amino]-phenol solution.

Analysis of the resulting precipitate showed a yield of 24.6 g (97.8 %) off-white powder of 2-(2-Benzothiazolyl)-5-[4,6-morpholin-s-triazin-2-yl]amino]-phenol. The compound demonstrated an maximum emission value at 590 nm (yellow-orange), a DTA peak of 289 °C, an intensity of fluorescence of 23 (1% draw down, [cd/m²]), and CIE color coordinates of 0.563 and 0.435 (X and Y, respectively).

### Example 8: Preparation of 2-(2-Benzothiazolyl)-5-[[4-chloro-6-piperidyl-s-triazin-2-yl] amino]-phenol

This example illustrates the preparation of the following fluorescent compound of the present invention:

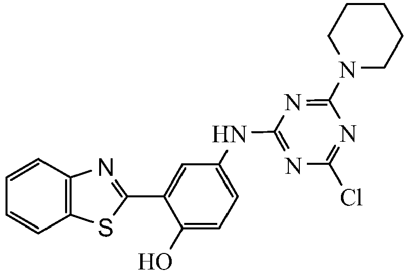

Approximately 10g (0.025 mol) of 2-(2-Benzothiazolyl)-5-[4,6-dichloro-s-triazin-2-yl]amino]-phenol was added to about 140 ml of butanone in a 1L three-neck round flask equipped with a drop funnel, mechanical stirrer and thermometer. Approximately 4.43 g (0.052 mol) of piperidine was added to the solution via the drop funnel over a period of approximately 15 min. The drop funnel was rinsed with 10 ml Butanone and the reaction mixture was stirred for 6 h at room temperature and then was left standing overnight at the same temperature. After this time the off-white precipitate was filtered by suction, washed with cold butanone and then two times suspended in 250 ml water. The solid was again filtered by suction and dried.

Analysis of the resulting precipitate showed a yield of 9.2 g (82.2%) sand-colored powder of 2-(2-Benzothiazolyl)-5-[4-chloro-6-piperidyl-s-triazin-2-yl]amino]-phenol. The compound demonstrated an maximum emission value at 560 nm (green-yellow), a DTA peak of 215 °C, an intensity of fluorescence of 55 (1% draw down, [cd/m²]), and CIE color coordinates of 0.490 and 0.505 (X and Y, respectively).

### Example 9: Preparation of 2-(2-Benzothiazolyl)-5-[[4-chloro-6-(4-methylpiperid-1-yl)-s-triazin-2-yl] amino]-phenol

This example illustrates the preparation of the following fluorescent compound of the present invention:

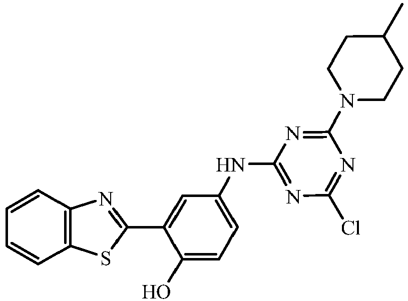

Approximately 10g (0.025 mol) of 2-(2-Benzothiazolyl)-5-[4,6-dichloro-s-triazin-2-yl]amino]-phenol was added to 250 ml 2-Butanone at room temperature for form a suspension. To this suspension about 5.17 g (0.0521 mol) of 4-Methylpiperidine was added. The mixture was stirred at room temperature for about 24 hrs. After this time the precipitate was filtered by suction and washed with 2-Butanone which resulted in the formation of sand-colored filtrates. About 2.5 g charcoal were added to the combined sand-colored filtrates and the resulting mixture was stirred for 4 hours at room temperature. Additional filtration resulted in a clear, slightly yellowish solution.

A drop on a filter paper gave a bright yellow fluorescence under 366 nm excitation. The resulting solution demonstrated an maximum emission value at 575 nm (yellow) and CIE color coordinates of 0.490 and 0.505 (X and Y, respectively).

### Example 10: Preparation of 2-(2-Benzothiazolyl)-5-[[4,6-bis(4-methylpiperid-1-yl)-s-triazin-2-yl] amino]-phenol

This example illustrates the preparation of the following fluorescent compound of the present invention:

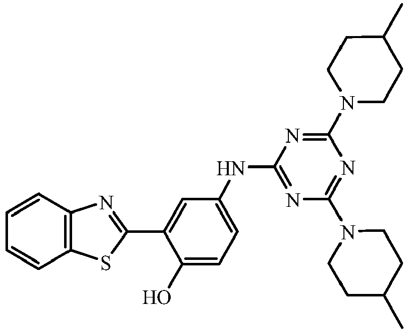

The procedure of Example 9 was repeated expect that 10.17 g (0.1025 mol) 4-methylpiperidine was added to the 2-(2-benzothiazolyl)-5-[4,6-dichloro-s-triazin-2-yl]amino]-phenol / 2-butanone suspension. The mixture was stirred at room temperature for about 24 hrs. After this time the precipitate was filtered by suction, washed with 2-butane, methanol, water and dried at 60°C.

Analysis of the resulting precipitate showed a yield of 9.2 g (70%) sand-colored powder of 2-(2-Benzothiazolyl)-5-[[4,6-bis(4-methylpiperid-1-yl)-s-triazin-2-yl]amino]-phenol. The compound demonstrated an maximum emission value at 580 nm (yellow), DTA peaks of 179 and 206 °C, an intensity of fluorescence of 19 (1% draw down, [cd/m²]), and CIE color coordinates of 0.533 and 0.464 (X and Y, respectively).

### Example 11: Preparation of 2-(2-Benzothiazolyl)-5-[[4,6-bis(4-methylpiperazin-1-yl)-s-triazin-2-yl] amino] -phenol

This example illustrates the preparation of the following fluorescent compound of the present invention:

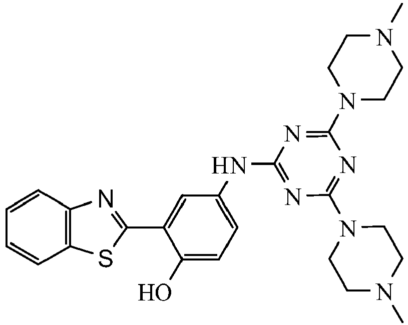

The procedure of Example 10 was repeated expect that instead of methylpiperidine, about 10.27 g (0.1025 mol) of 4-Methylpiperazine was used.

Analysis of the resulting precipitate showed a yield of 12.5 g (94.6 %) of a yellow-beige colored fine powder of 2-(2-Benzothiazolyl)-5-[[4,6-bis(4-methylpiperazin-1-yl)-s-triazin-2-yl]amino]-phenol. The compound demonstrated an maximum emission value at 565 nm (yellow-green), a DTA peak of 214 °C, an intensity of fluorescence of 29 (1% draw down, [cd/m²]), and CIE color coordinates of 0.536 and 0.461 (X and Y, respectively).

### Example 12: Preparation of 1,3-bis[[3-(2-Benzothiazolyl)-4-phenyl]-amino]-5-chloro-triazine

This example illustrates the preparation of the following fluorescent compound of the present invention:

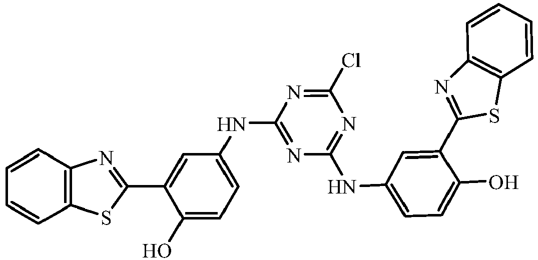

About 3.9 g (0.01 mol) of 2-(2-Benzothiazolyl)-5-[4,6-dichloro-s-triazin-2-yl]amino]-phenol, 2.42 g (0.01 mol) of (hydroxy-5-aminophenyl)-benzthiazole, and 0.8 g of sodium bicarbonate were added to 50 ml 2-butanone to produce a suspension. The suspension was stirred while heated to between 50 - 60 °C. The suspension became bright beige. After two hours the mixture was cooled to room temperature and filtered by suction. The product was stirred in 100 ml water at room temperature for 15 min, filtered again by suction, and then dried at 85 °C resulting in a grey fine powder with yellow fluorescence.

Analysis of the resulting precipitate showed a yield of 5.96 g (69.8 %) of a yellow-beige colored fine powder of 1,3-bis[[3-(2-Benzothiazolyl)-4-phenyl]-amino]-5-chloro-triazine. The compound demonstrated an maximum emission value at 565 nm (yellow-green), a DTA peak of 302 °C, an intensity of fluorescence of 68 (1 % draw down, [cd/m²]), and CIE color coordinates of 0.494 and 0.500 (X and Y, respectively).

### Example 13: Preparation of 1,3-bis[[3-(2-Benzothiazolyl)-4-phenyl]-amino]-5-morpholin-triazine

This example illustrates the preparation of the following fluorescent compound of the present invention:

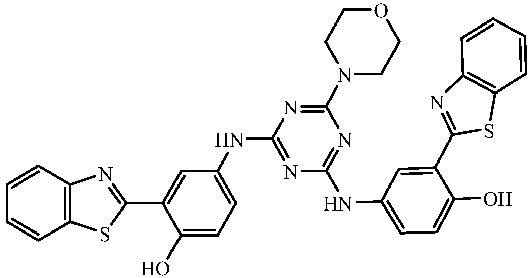

Approximately 2 g (0.003 mol) of the 1,3-bis[[3-(2-Benzothiazolyl)-4-phenyl]-amino]-5-chloro-triazine precipitate from Example 12 was added to 50 ml Butanone and stirred to produce a suspension. About 0.8 ml Morpholine was added to this suspension and then the mixture was heated to reflux for 2 hrs. After cooling to room temperature the product was filtered by suction, washed with 100 ml Butane, then with 100 ml acetone, and then with 100 ml water. The washed product was then dried at 85 °C resulting in a yellowish powder with yellow fluorescence.

Analysis of the resulting precipitate showed a yield of 1.99 g (92 %) of a yellow-colored fine powder of 1,3-bis[[3-(2-Benzothiazolyl)-4-phenyl]-amino]-5-morpholin-triazine. The compound demonstrated an maximum emission value at 570 nm (yellow), a DTA peak of 298 °C, an intensity of fluorescence of 74 (1 % draw down, [cd/m²]), and CIE color coordinates of 0.517 and 0.479 (X and Y, respectively).

## Claims

1. A composition comprising an organic fluorophore having a structure according to Formula I: 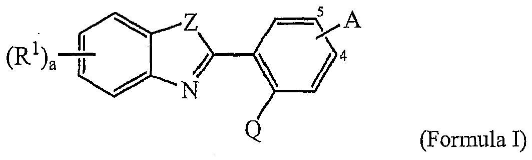 wherein:
R¹ is independently a C₁ - C₈ branched or straight chain alkyl;
a is 0, 1, 2, 3, or 4;
Z is S;
Q is an acidic hydrogen moiety selected from OH, NH₂, NHR², wherein R² is a C₁ - C₈ branched or straight chain alkyl; and
A is an amino-triazine moiety bonded to the 4^{th} or 5^{th} carbon of the phenol or aniline ring, said amino-triazine moiety having a structure according to Formula II: 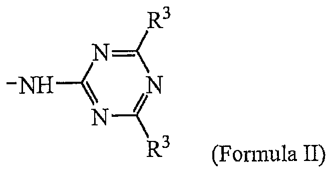
wherein R³ is independently
a halogen,
a benzothiazole having a structure according to Formula X: 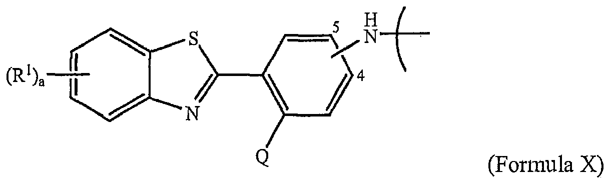
wherein Q, R¹, and a are defined as above,
a benzooxazole having a structure according to Formula XI: 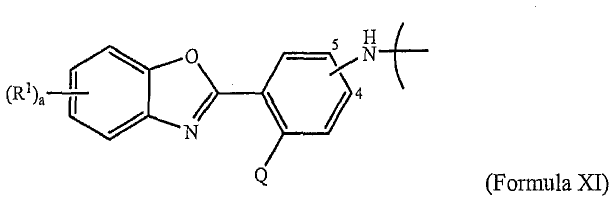
wherein Q, R¹, and a are defined as above,
or a tertiary amine having a structure according to Formula III: 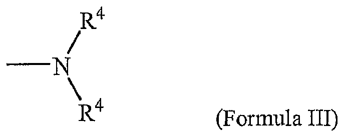
wherein R⁴ is independently a C₁ - C₂₀ linear or branched, substituted or unsubstituted alkyl, alkyoxy, hydroxyalky, or carboxylic acid, or a member of substituted or unsubstituted C₁ - C₂₀ heterocyclic alkyl having the N atom positioned between the two R⁴ groups, and optionally having additional N, O, or S heteroatoms;
provided that no more than one R³ is a halogen and that no more than one R³ is said benzothiazole or said benzooxazole.

2. The composition of claim 1 wherein said organic fluorophore is a fluorescent pigment and R³ is independently chlorine, substituted or unsubstituted piperidyl, substituted or unsubstituted pyrrolidyl, substituted or unsubstituted piperazin-1-yl, substituted or unsubstituted morpholin-4-yl, provided that no more than one R³ is chlorine.

3. The composition of claim 1 wherein one R³ is

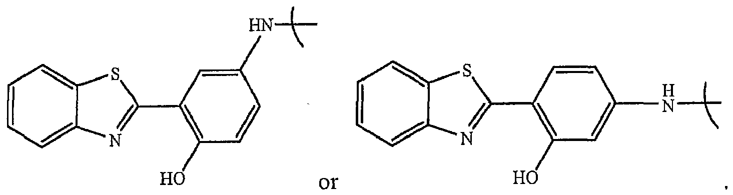

4. A fluorescent compound having a structure according to Formulae IV or V: 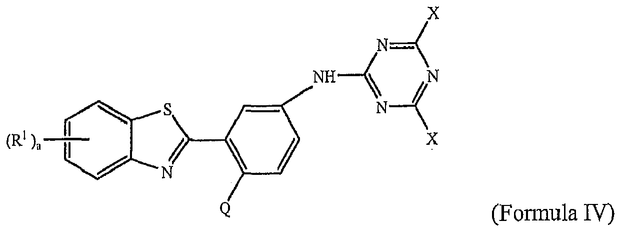 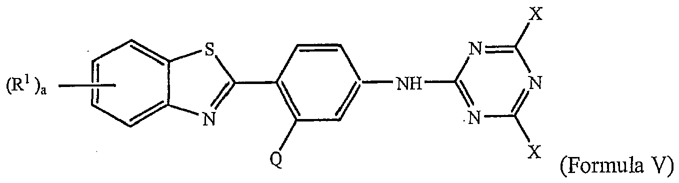 wherein:
R¹ is independently a C₁ - C₈ branched or straight chain alkyl;
a is 0, 1, 2, 3, or 4;
Q is an acidic hydrogen moiety selected from OH, NH₂, NHR², wherein R² is a C₁ - C₈ branched or straight chain alkyl; and
X is independently a halogen.

5. The compound of claim 4 wherein X is Cl

6. The compound of claim 5 wherein a is 0.

7. The compound of claim 6 wherein Q is OH.

8. An article of manufacture comprising a substrate selected from synthetic polymeric fibers, cellulose, and cellulose derivatives covalently bonded to an organic fluorophore having a structure according to Formula VI; 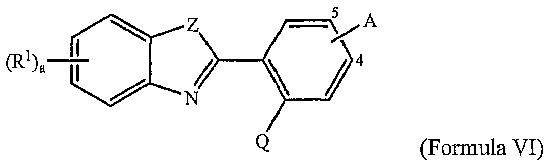 wherein:
R¹ is independently a C₁ - C₈ branched or straight chain alkyl;
a is 0, 1, 2, 3, or 4;
Z is O or S;
Q is an acidic hydrogen moiety selected from OH, NH₂, NHR², wherein R² is a C₁ - C₈ branched or straight chain alkyl; and
A is an amino-triazine moiety bonded to the 4^{th} or 5^{th} carbon of the phenol or aniline ring, said amino-triazine moiety having a structure according to Formula VII: 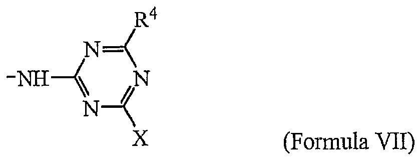
wherein X is a halogen and R⁴ is a covalent bond joining said organic fluorophore and said substrate.

9. A method for producing a fluorescent fiber comprising;
a. providing a cellulose or cellulose derivative fiber, and
b. contacting the fiber with a fluorescent compound under conditions effective to covalently bond the fluorescent compound to the fiber, wherein the fluorescent compound has a structure according to Formula VIII: 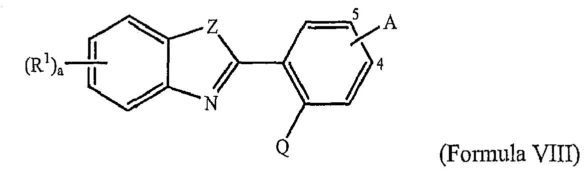 wherein:
R¹ is independently a C₁- C₈ branched or straight chain alkyl;
a is 0, 1, 2, 3, or 4;
Z is O or S;
Q is an acidic hydrogen moiety selected from OH, NH₂, NHR², wherein R² is a C₁ - C₈ branched or straight chain alkyl; and
A is an amino-triazine moiety bonded to the 4^{th} or 5^{th} carbon of the phenol or aniline ring, said amino-triazine moiety having a structure according to Formula IX: 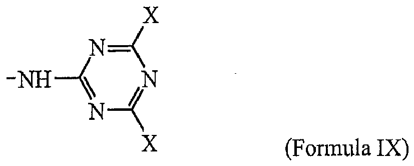 wherein X is independently a halogen.

10. A method for marking an article comprising contacting at least a portion of said article with a composition comprising an organic fluorophore having a structure according to Formula I 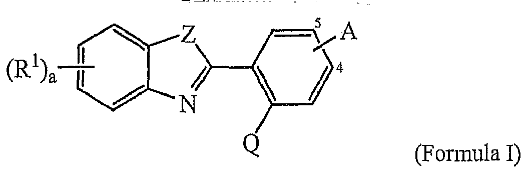 wherein:
R¹ is independently a C₁ - C₈ branched or straight chain alkyl;
a is 0, 1, 2, 3, or 4;
Z is O or S;
Q is an acidic hydrogen moiety selected from OH, NH₂, NHR², wherein R² is a C₁-C₈ branched or straight chain alkyl; and
A is an amino-triazine moiety bonded to the 4^{th} or 5^{th} carbon of the phenol or aniline ring, said amino-triazine moiety having a structure according to Formula II: 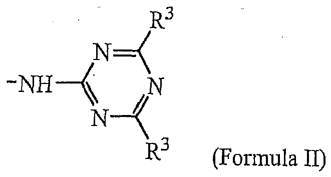
wherein R³ is independently
a halogen,
a benzothiazole having a structure according to Formula X: 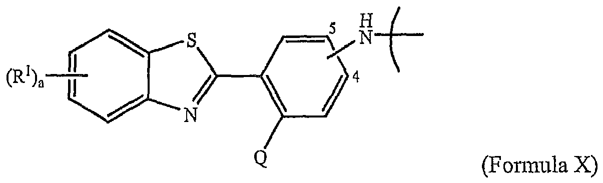
wherein Q, R¹, and a are defined as above,
a benzooxazole having a structure according to Formula XI: 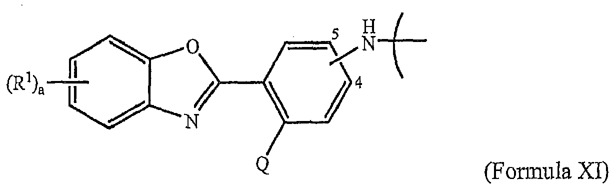
wherein Q, R¹, and a are defined as above,
or a tertiary amine having a structure according to Formula III: 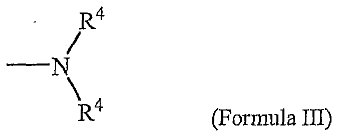
wherein R⁴ is independently a C₁ - C₂₀ linear or branched, substituted or unsubstituted alkyl, alkyoxy, hydroxyalky, or carboxylic acid, or a member of substituted or unsubstituted C₁ - C₂₀ heterocyclic alkyl having the N atom positioned between the two R⁴ groups, and optionally having additional N, O, or S heteroatoms;
provided that no more than one R³ is a halogen and that no more than one R³ is said benzothiazole or said benzooxazole
wherein said contacting produces a detectible residue on said portion of said article and wherein said article comprises a substrate selected from synthetic polymeric fibres, cellulose and cellulose derivatives.

11. A method according to claim 10, wherein the article is selected from an identity card, bank note, voucher, ticket, check coupon, tag, label, certificate and metering application.

12. Use of a fluorophore having a structure according to Formula I 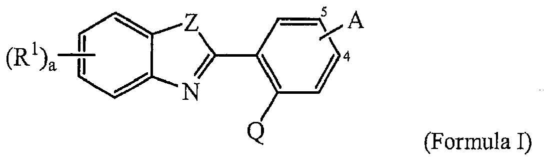 wherein:
R¹ is independently a C₁ - C₈ branched or straight chain alkyl;
a is 0, 1, 2, 3, or 4;
Z is O or S;
Q is an acidic hydrogen moiety selected from OH, NH₂, NHR², wherein R² is a C₁ - C₈ branched or straight chain alkyl; and
A is an amino-triazine moiety bonded to the 4^{th} or 5^{th} carbon of the phenol or aniline ring, said amino-triazine moiety having a structure according to Formula II: 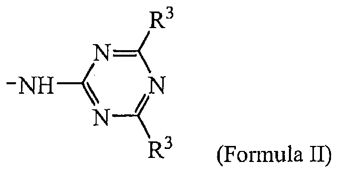
wherein R³ is independently
a halogen,
a benzothiazole having a structure according to Formula X: 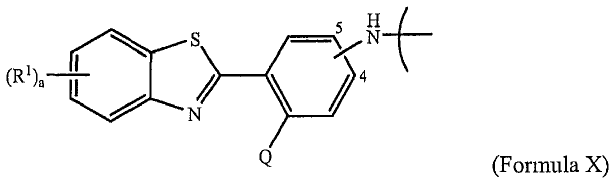
wherein Q, R¹, and a are defined as above,
a benzooxazole having a structure according to Formula XI: 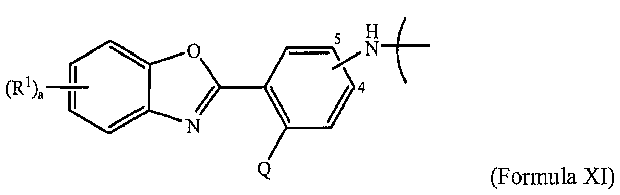
wherein Q, R¹, and a are defined as above,
or a tertiary amine having a structure according to Formula III: 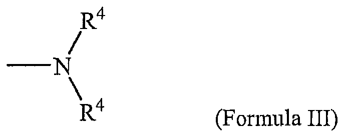
wherein R⁴ is independently a C₁ - C₂₀ linear or branched, substituted or unsubstituted alkyl, alkyoxy, hydroxyalky, or carboxylic acid, or a member of substituted or unsubstituted C₁ - C₂₀ heterocyclic alkyl having the N atom positioned between the two R⁴ groups, and optionally having additional N, O, or S heteroatoms;
provided that no more than one R³ is a halogen and that no more than one R³ is said benzothiazole or said benzooxazole as a security marker.

## Patentansprüche

1. Zusammensetzung, umfassend ein organisches Fluorophor mit einer Struktur gemäß Formel I 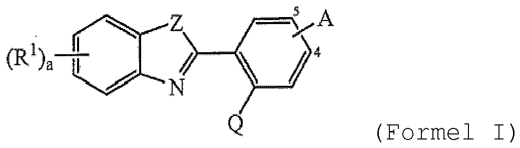 worin:
R¹ unabhängig für ein verzweigtes oder geradkettiges C₁-C₈-Alkyl steht;
a für 0, 1, 2, 3 oder 4 steht;
Z für S steht;
Q für eine sauren Wasserstoff enthaltende Gruppierung steht, die aus OH, NH₂ und NHR², worin R² für ein verzweigtes oder geradkettiges C₁-C₈-Alkyl steht, ausgewählt ist; und
A für eine Aminotriazingruppierung steht, die an den 4. oder 5. Kohlenstoff des Phenol- bzw. Anilinrings gebunden ist und eine Struktur gemäß Formel II aufweist: 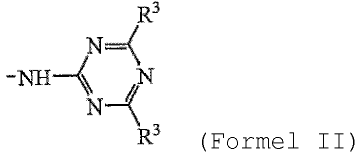
worin R³ unabhängig für
ein Halogen,
ein Benzothiazol mit einer Struktur gemäß Formel X: 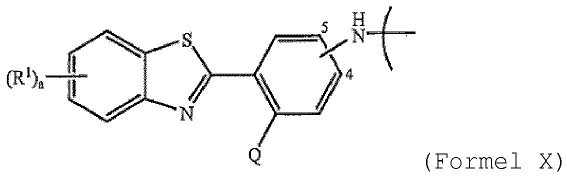
worin Q, R¹ und a wie oben definiert sind,
ein Benzooxazol mit einer Struktur gemäß Formel XI: 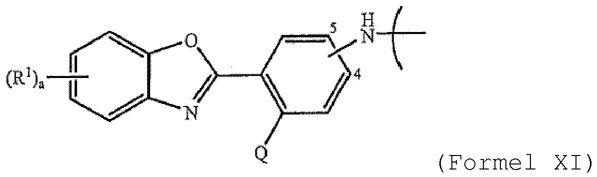
worin Q, R¹ und a wie oben definiert sind,
oder ein tertiäres Amin mit einer Struktur gemäß Formel III: 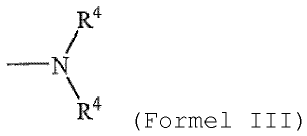
worin R⁴ unabhängig für ein lineares oder verzweigtes, substituiertes oder unsubstituiertes C₁-C₂₀-Alkyl, ein lineares oder verzweigtes, substituiertes oder unsubstituiertes C₁-C₂₀-Alkoxy, ein lineares oder verzweigtes, substituiertes oder unsubstituiertes C₁-C₂₀-Hydroxy-alkyl oder eine lineare oder verzweigte, substituierte oder unsubstituierte C₁-C₂₀-Carbonsäure oder ein Mitglied von substituiertem oder unsubstituiertem heterocyclischem C₁-C₂₀-Alkyl mit dem N-Atom zwischen den beiden R⁴-Gruppen, das gegebenenfalls zusätzliche N-, O- oder S-Heteroatome aufweist,
steht; mit der Maßgabe, dass höchstens ein R³ für ein Halogen steht und dass höchstens ein R³ für das Benzothiazol oder das Benzooxazol steht.

2. Zusammensetzung nach Anspruch 1, wobei es sich bei dem organischen Fluorophor um ein fluoreszierendes Pigment handelt und R³ unabhängig für Chlor, substituiertes oder unsubstituiertes Piperidyl, substituiertes oder unsubstituiertes Pyrrolidyl, substituiertes oder unsubstituiertes Piperazin-1-yl oder substituiertes oder unsubstituiertes Morpholin-4-yl steht, mit der Maßgabe, dass höchstens ein R³ für Chlor steht.

3. Zusammensetzung nach Anspruch 1, wobei ein R³ für 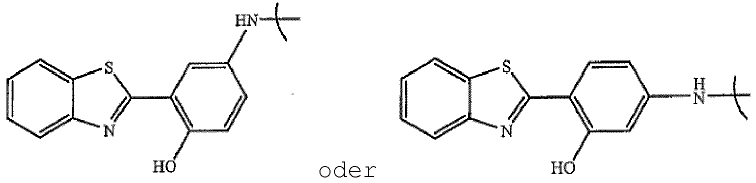 steht.

4. Fluoreszierende Verbindung mit einer Struktur gemäß Formel IV oder V: 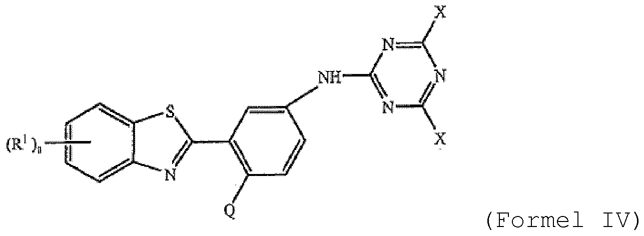 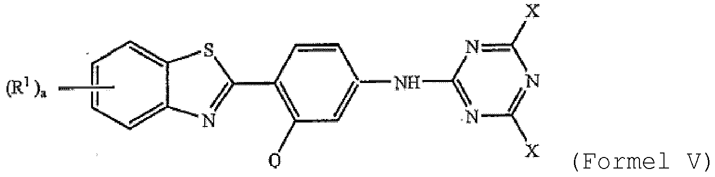 worin:
R¹ unabhängig für ein verzweigtes oder geradkettiges C₁-C₈-Alkyl steht;
a für 0, 1, 2, 3 oder 4 steht;
Q für eine sauren Wasserstoff enthaltende Gruppierung steht, die aus OH, NH₂ und NHR², worin R² für ein verzweigtes oder geradkettiges C₁-C₈-Alkyl steht, ausgewählt ist; und
X unabhängig für ein Halogen steht.

5. Verbindung nach Anspruch 4, wobei X für Cl steht.

6. Verbindung nach Anspruch 5, wobei a für 0 steht.

7. Verbindung nach 6, wobei Q für OH steht.

8. Erzeugnis, umfassend ein aus Kunststofffasern, Cellulose und Cellulosederivaten ausgewähltes Substrat, das kovalent an ein organisches Fluorophor mit einer Struktur gemäß Formel VI gebunden ist: 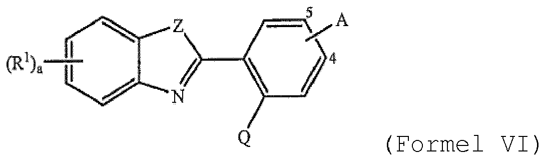 worin:
R¹ unabhängig für ein verzweigtes oder geradkettiges C₁-C₈-Alkyl steht;
a für 0, 1, 2, 3 oder 4 steht;
Z für O oder S steht;
Q für eine sauren Wasserstoff enthaltende Gruppierung steht, die aus OH, NH₂ und NHR², worin R² für ein verzweigtes oder geradkettiges C₁-C₈-Alkyl steht, ausgewählt ist; und
A für eine Aminotriazingruppierung steht, die an den 4. oder 5. Kohlenstoff des Phenol- bzw. Anilinrings gebunden ist und eine Struktur gemäß Formel VII aufweist: 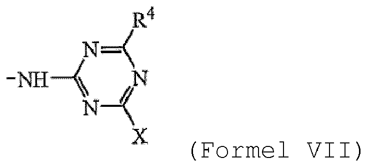
worin X für ein Halogen steht und R⁴ für eine kovalente Bindung, die das organische Fluorophor und das Substrat verknüpft, steht.

9. Verfahren zur Herstellung einer fluoreszierenden Faser, umfassend:
a. Bereitstellen einer Cellulose- oder Cellulosederivatfaser und
b. Inberührungbringen der Faser mit einer fluoreszierenden Verbindung unter Bedingungen, unter denen die fluoreszierende Verbindung kovalent an die Faser gebunden wird, wobei die fluoreszierende Verbindung eine Struktur der Formel VIII aufweist: 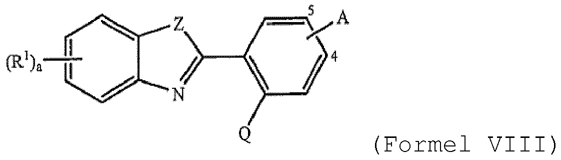 worin:
R¹ unabhängig für ein verzweigtes oder geradkettiges C₁-C₈-Alkyl steht;
a für 0, 1, 2, 3 oder 4 steht;
Z für O oder S steht;
Q für eine sauren Wasserstoff enthaltende Gruppierung steht, die aus OH, NH₂ und NHR², worin R² für ein verzweigtes oder geradkettiges C₁-C₈-Alkyl steht, ausgewählt ist; und
A für eine Aminotriazingruppierung steht, die an den 4. oder 5. Kohlenstoff des Phenol- bzw. Anilinrings gebunden ist und eine Struktur gemäß Formel IX aufweist: 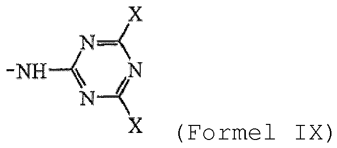
worin X unabhängig für ein Halogen steht.

10. Verfahren zur Markierung eines Gegenstands, umfassend das Inberührungbringen mindestens eines Teils des Gegenstands mit einer Zusammensetzung, umfassend ein organisches Fluorophor mit einer Struktur gemäß Formel I 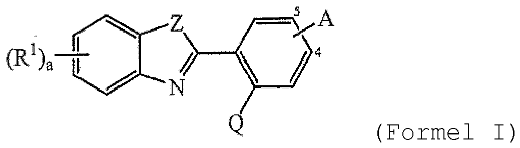 worin:
R¹ unabhängig für ein verzweigtes oder geradkettiges C₁-C₈-Alkyl steht;
a für 0, 1, 2, 3 oder 4 steht;
Z für O oder S steht;
Q für eine sauren Wasserstoff enthaltende Gruppierung steht, die aus OH, NH₂ und NHR², worin R² für ein verzweigtes oder geradkettiges C₁-C₈-Alkyl steht, ausgewählt ist; und
A für eine Aminotriazingruppierung steht, die an den 4. oder 5. Kohlenstoff des Phenol- bzw. Anilinrings gebunden ist und eine Struktur gemäß Formel II aufweist: 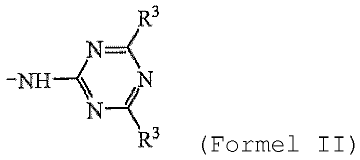
worin R³ unabhängig für
ein Halogen,
ein Benzothiazol mit einer Struktur gemäß Formel X: 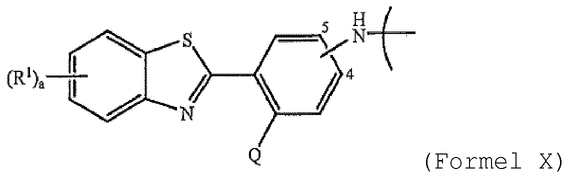
worin Q, R¹ und a wie oben definiert sind,
ein Benzooxazol mit einer Struktur gemäß Formel XI: 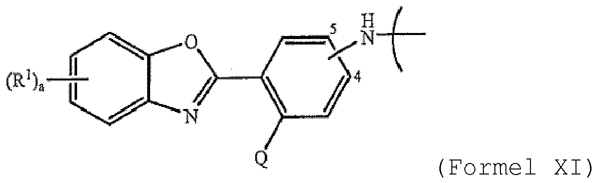
worin Q, R¹ und a wie oben definiert sind,
oder ein tertiäres Amin mit einer Struktur gemäß Formel III: 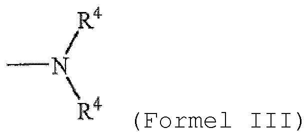
worin R⁴ unabhängig für ein lineares oder verzweigtes, substituiertes oder unsubstituiertes C₁-C₂₀-Alkyl, ein lineares oder verzweigtes, substituiertes oder unsubstituiertes C₁-C₂₀-Alkoxy, ein lineares oder verzweigtes, substituiertes oder unsubstituiertes C₁-C₂₀-Hydroxy-alkyl oder eine lineare oder verzweigte, substituierte oder unsubstituierte C₁-C₂₀-Carbonsäure oder ein Mitglied von substituiertem oder unsubstituiertem heterocyclischem C₁-C₂₀-Alkyl mit dem N-Atom zwischen den beiden R⁴-Gruppen, das gegebenenfalls zusätzliche N-, O-oder S-Heteroatome aufweist,
steht; mit der Maßgabe, dass höchstens ein R³ für ein Halogen steht und dass höchstens ein R³ für das Benzothiazol oder das Benzooxazol steht;
wobei das Inberührungbringen einen nachweisbaren Rückstand auf dem Teil des Gegenstands erzeugt und wobei der Gegenstand ein aus Kunststofffasern, Cellulose und Cellulosederivaten ausgewähltes Substrat umfasst.

11. Verfahren nach Anspruch 10, bei dem der Gegenstand aus einem Personalausweis, einer Banknote, einem Gutschein, einem Ticket, einem Scheck, einem Coupon, einer Markierung, einem Etikett, einer Urkunde und einer Messanwendung ausgewählt ist.

12. Verwendung eines Fluorophors mit einer Struktur gemäß Formel I 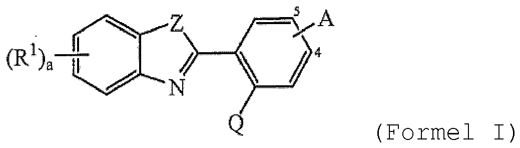 worin:
R¹ unabhängig für ein verzweigtes oder geradkettiges C₁-C₈-Alkyl steht;
a für 0, 1, 2, 3 oder 4 steht;
Z für O oder S steht;
Q für eine sauren Wasserstoff enthaltende Gruppierung steht, die aus OH, NH₂ und NHR², worin R² für ein verzweigtes oder geradkettiges C₁-C₈-Alkyl steht, ausgewählt ist; und
A für eine Aminotriazingruppierung steht, die an den 4. oder 5. Kohlenstoff des Phenol- bzw. Anilinrings gebunden ist und eine Struktur gemäß Formel II aufweist: 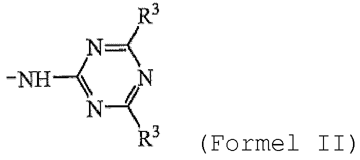
worin R³ unabhängig für
ein Halogen,
ein Benzothiazol mit einer Struktur gemäß Formel X: 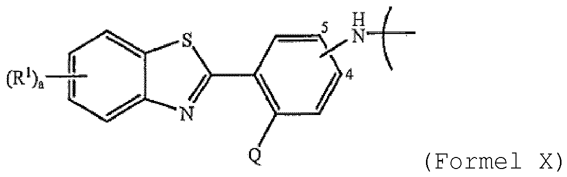
worin Q, R¹ und a wie oben definiert sind,
ein Benzooxazol mit einer Struktur gemäß Formel XI: 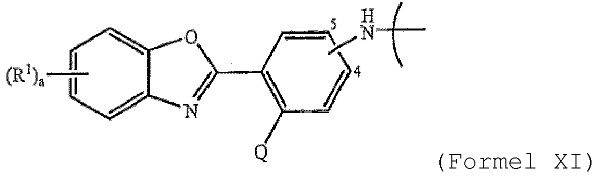
worin Q, R¹ und a wie oben definiert sind,
oder ein tertiäres Amin mit einer Struktur gemäß Formel III: 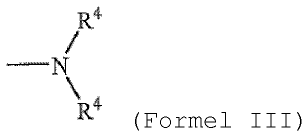
worin R⁴ unabhängig für ein lineares oder verzweigtes, substituiertes oder unsubstituiertes C₁-C₂₀-Alkyl, ein lineares oder verzweigtes, substituiertes oder unsubstituiertes C₁-C₂₀-Alkoxy, ein lineares oder verzweigtes, substituiertes oder unsubstituiertes C₁-C₂₀-Hydroxy-alkyl oder eine lineare oder verzweigte, substituierte oder unsubstituierte C₁-C₂₀-Carbonsäure oder ein Mitglied von substituiertem oder unsubstituiertem heterocyclischem C₁-C₂₀-Alkyl mit dem N-Atom zwischen den beiden R⁴-Gruppen, das gegebenenfalls zusätzliche N-, O-oder S-Heteroatome aufweist,
steht; mit der Maßgabe, dass höchstens ein R³ für ein Halogen steht und dass höchstens ein R³ für das Benzothiazol oder das Benzooxazol steht;
als Sicherheitsmarkierung.

## Revendications

1. Composition comprenant un fluorophore organique ayant une structure répondant à la formule I : 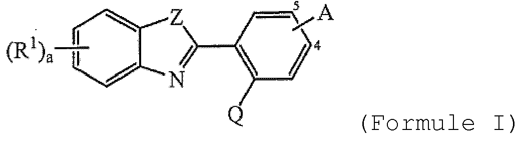 dans laquelle :
R¹ représente indépendamment un alkyle en C₁-C₈ à chaîne ramifiée ou linéaire ;
a vaut 0, 1, 2, 3 ou 4 ;
Z représente S ;
Q représente une fraction à hydrogène acide choisie parmi OH, NH₂ et NHR² où R² représente un alkyle en C₁-C₈ à chaîne ramifiée ou linéaire ; et
A représente une fraction amino-triazine liée au 4^{e} ou 5^{e} carbone du cycle phénol ou aniline, ladite fraction amino-triazine ayant une structure répondant à la formule II : 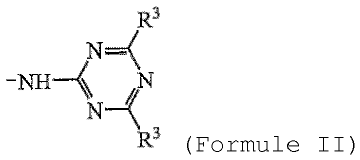
dans laquelle R³ représente indépendamment
un halogène,
un benzothiazole ayant une structure répondant à la formule X : 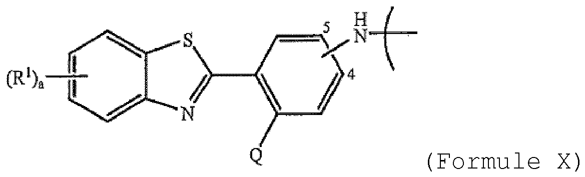
dans laquelle Q, R¹ et a sont définis comme ci-dessus,
un benzooxazole ayant une structure répondant à la formule XI : 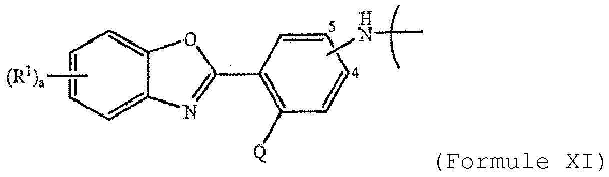
dans laquelle Q, R¹ et a sont définis comme ci-dessus,
ou une amine tertiaire ayant une structure répondant à la formule III : 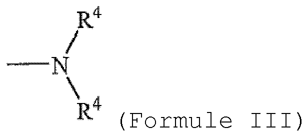
dans laquelle R⁴ représente indépendamment un groupe alkyle, alcoxy, hydroxyalkyle ou acide carboxylique en C₁-C₂₀ linéaire ou ramifié, substitué ou non substitué, ou un élément d'alkyle hétérocyclique en C₁-C₂₀ substitué ou non substitué ayant l'atome N positionné entre les deux groupes R⁴, et ayant éventuellement des hétéroatomes N, O ou S supplémentaires ;
à condition que pas plus d'un R³ représente un halogène et pas plus d'un R³ représente ledit benzothiazole ou ledit benzooxazole.

2. Composition selon la revendication 1 dans laquelle ledit fluorophore organique est un pigment fluorescent et R³ représente indépendamment un chlore ou un groupe pipéridyle substitué ou non substitué, pyrrolidyle substitué ou non substitué, pipérazin-1-yle substitué ou non substitué, ou morpholin-4-yle substitué ou non substitué, à condition que pas plus d'un R³ représente un chlore.

3. Composition selon la revendication 1 dans laquelle un R³ représente

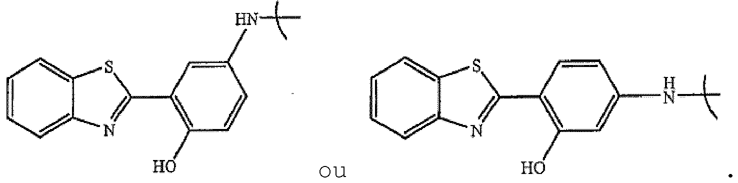

4. Composé fluorescent ayant une structure répondant aux formules IV ou V : 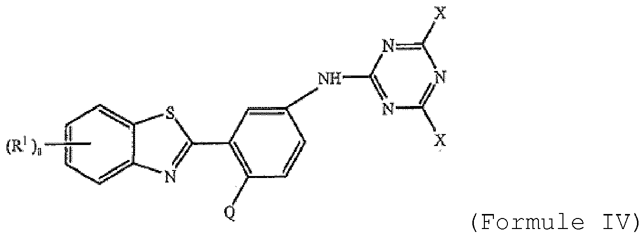 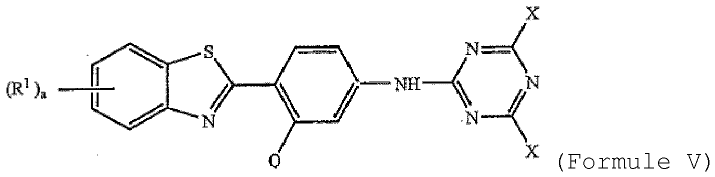 dans lesquelles :
R¹ représente indépendamment un alkyle en C₁-C₈ à chaîne ramifiée ou linéaire ;
a vaut 0, 1, 2, 3 ou 4 ;
Q représente une fraction à hydrogène acide choisie parmi OH, NH₂ et NHR² où R² représente un alkyle en C₁-C₈ à chaîne ramifiée ou linéaire ; et
X représente indépendamment un halogène.

5. Composé selon la revendication 4 dans lequel X représente Cl.

6. Composé selon la revendication 5 dans lequel a vaut 0.

7. Composé selon la revendication 6 dans lequel Q représente OH.

8. Article de fabrication comprenant un substrat choisi parmi les fibres polymères synthétiques, la cellulose et les dérivés de cellulose, lié de façon covalente à un fluorophore organique ayant une structure répondant à la formule VI : 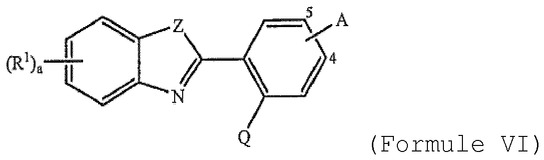 dans laquelle :
R¹ représente indépendamment un alkyle en C₁-C₈ à chaîne ramifiée ou linéaire ;
a vaut 0, 1, 2, 3 ou 4 ;
Z représente O ou S ;
Q représente une fraction à hydrogène acide choisie parmi OH, NH₂ et NHR² où R² représente un alkyle en C₁-C₈ à chaîne ramifiée ou linéaire ; et
A représente une fraction amino-triazine liée au 4^{e} ou 5^{e} carbone du cycle phénol ou aniline, ladite fraction amino-triazine ayant une structure répondant à la formule VII : 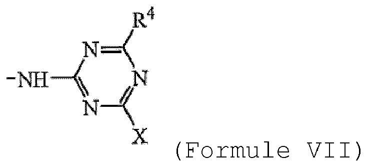
dans laquelle X représente un halogène et R⁴ représente une liaison covalente liant ledit fluorophore organique et ledit substrat.

9. Procédé de production d'une fibre fluorescente comprenant :
a. l'obtention d'une fibre de cellulose ou de dérivé de cellulose, et
b. la mise en contact de la fibre avec un composé fluorescent dans des conditions efficaces pour lier de façon covalente le composé fluorescent à la fibre, le composé fluorescent ayant une structure répondant à la formule VIII : 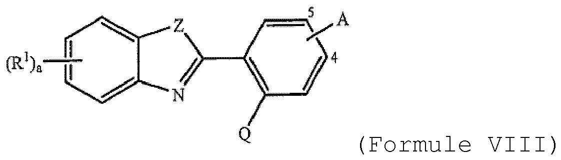 dans laquelle :
R¹ représente indépendamment un alkyle en C₁-C₈ à chaîne ramifiée ou linéaire ;
a vaut 0, 1, 2, 3 ou 4 ;
Z représente O ou S ;
Q représente une fraction à hydrogène acide choisie parmi OH, NH₂ et NHR² où R² représente un alkyle en C₁-C₈ à chaîne ramifiée ou linéaire ; et
A représente une fraction amino-triazine liée au 4^{e} ou 5^{e} carbone du cycle phénol ou aniline, ladite fraction amino-triazine ayant une structure répondant à la formule IX : 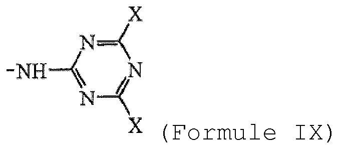
dans laquelle X représente indépendamment un halogène.

10. Procédé de marquage d'un article comprenant la mise en contact d'au moins une partie dudit article avec une composition comprenant un fluorophore organique ayant une structure répondant à la formule I : 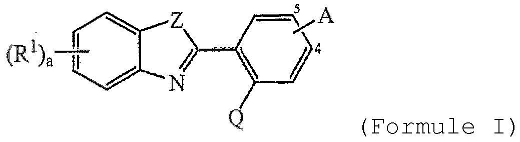 dans laquelle :
R¹ représente indépendamment un alkyle en C₁-C₈ à chaîne ramifiée ou linéaire ;
a vaut 0, 1, 2, 3 ou 4 ;
Z représente O ou S ;
Q représente une fraction à hydrogène acide choisie parmi OH, NH₂ et NHR² où R² représente un alkyle en C₁-C₈ à chaîne ramifiée ou linéaire ; et
A représente une fraction amino-triazine liée au 4^{e} ou 5^{e} carbone du cycle phénol ou aniline, ladite fraction amino-triazine ayant une structure répondant à la formule II : 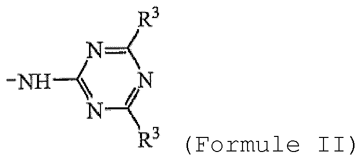
dans laquelle R³ représente indépendamment
un halogène,
un benzothiazole ayant une structure répondant à la formule X : 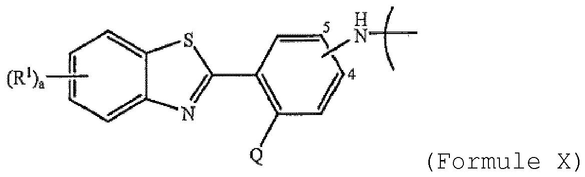
dans laquelle Q, R¹ et a sont définis comme ci-dessus,
un benzooxazole ayant une structure répondant à la formule XI : 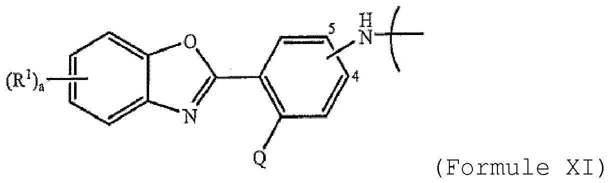
dans laquelle Q, R¹ et a sont définis comme ci-dessus,
ou une amine tertiaire ayant une structure répondant à la formule III : 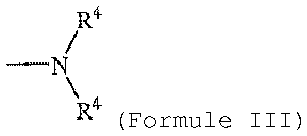
dans laquelle R⁴ représente indépendamment un groupe alkyle, alcoxy, hydroxyalkyle ou acide carboxylique en C₁-C₂₀ linéaire ou ramifié, substitué ou non substitué, ou un élément d'alkyle hétérocyclique en C₁-C₂₀ substitué ou non substitué ayant l'atome N positionné entre les deux groupes R⁴, et ayant éventuellement des hétéroatomes N, O ou S supplémentaires ;
à condition que pas plus d'un R³ représente un halogène et pas plus d'un R³ représente ledit benzothiazole ou ledit benzooxazole
dans lequel ladite mise en contact produit un résidu détectable sur ladite partie dudit article et
dans lequel ledit article comprend un substrat choisi parmi les fibres polymères synthétiques, la cellulose et les dérivés de cellulose.

11. Procédé selon la revendication 10, dans lequel l'article est choisi parmi une carte d'identité, un billet de banque, un bon, un ticket, un coupon de chèque, une étiquette, une vignette, un certificat et une application de mesure.

12. Utilisation d'un fluorophore ayant une structure répondant à la formule I : 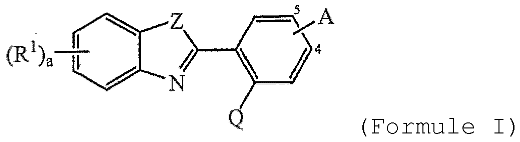 dans laquelle :
R¹ représente indépendamment un alkyle en C₁-C₈ à chaîne ramifiée ou linéaire ;
a vaut 0, 1, 2, 3 ou 4 ;
Z représente O ou S ;
Q représente une fraction à hydrogène acide choisie parmi OH, NH₂ et NHR² où R² représente un alkyle en C₁-C₈ à chaîne ramifiée ou linéaire ; et
A représente une fraction amino-triazine liée au 4^{e} ou 5^{e} carbone du cycle phénol ou aniline, ladite fraction amino-triazine ayant une structure répondant à la formule II : 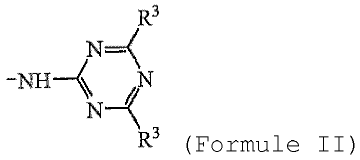
dans laquelle R³ représente indépendamment
un halogène,
un benzothiazole ayant une structure répondant à la formule X : 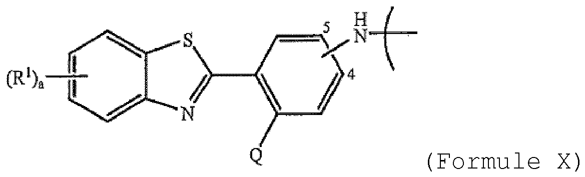
dans laquelle Q, R¹ et a sont définis comme ci-dessus,
un benzooxazole ayant une structure répondant à la formule XI : 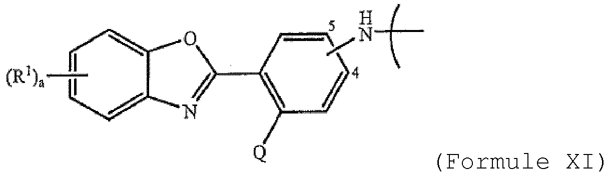
dans laquelle Q, R¹ et a sont définis comme ci-dessus,
ou une amine tertiaire ayant une structure répondant à la formule III : 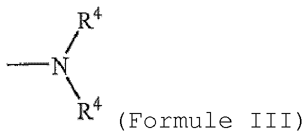
dans laquelle R⁴ représente indépendamment un groupe alkyle, alcoxy, hydroxyalkyle ou acide carboxylique en C₁-C₂₀ linéaire ou ramifié, substitué ou non substitué, ou un élément d'alkyle hétérocyclique en C₁-C₂₀ substitué ou non substitué ayant l'atome N positionné entre les deux groupes R⁴, et ayant éventuellement des hétéroatomes N, O ou S supplémentaires ;
à condition que pas plus d'un R³ représente un halogène et pas plus d'un R³ représente ledit benzothiazole ou ledit benzooxazole
comme marqueur de sécurité.
